(19) **Europäisches Patentamt** / **European Patent Office** / **Office européen des brevets**

(11) **EP 3 718 060 B1**

(12) # EUROPEAN PATENT SPECIFICATION

(45) Date of publication and mention of the grant of the patent:
**20.08.2025 Bulletin 2025/34**

(21) Application number: **18882806.5**

(22) Date of filing: **30.11.2018**

(51) International Patent Classification (IPC):
**G16C 10/00** *(2019.01)*     **G16C 20/90** *(2019.01)*
**G06N 5/01** *(2023.01)*     **G06N 10/20** *(2022.01)*
**G16C 20/30** *(2019.01)*

(52) Cooperative Patent Classification (CPC):
**G16C 10/00; G06N 5/01; G06N 10/20; G16C 20/90; G16C 20/30**

(86) International application number:
**PCT/CA2018/051531**

(87) International publication number:
**WO 2019/104440 (06.06.2019 Gazette 2019/23)**

(54) **METHODS AND SYSTEMS FOR QUANTUM COMPUTING ENABLED MOLECULAR AB INITIO SIMULATIONS USING QUANTUM-CLASSICAL COMPUTING HARDWARE**

VERFAHREN UND SYSTEME FÜR DURCH QUANTEN-COMPUTING ERMÖGLICHTE MOLEKULARE AB-INITIO-SIMULATIONEN UNTER VERWENDUNG VON QUANTEN-KLASSISCHER COMPUTING-HARDWARE

PROCÉDÉS ET SYSTÈMES POUR DES SIMULATIONS D'AB INITIO MOLÉCULAIRES ACTIVÉES PAR UN CALCUL QUANTIQUE FAISANT INTERVENIR UN MATÉRIEL INFORMATIQUE CLASSIQUE QUANTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2017 US 201762593060 P**

(43) Date of publication of application:
**07.10.2020 Bulletin 2020/41**

(73) Proprietor: **Good Chemistry Inc.**
**Vancouver BC V6C 0A3 (CA)**

(72) Inventors:
- **YAMAZAKI, Takeshi**
  **Vancouver**
  **British Columbia V5R 6C5 (CA)**
- **ZARIBAFIYAN, Arman**
  **Vancouver**
  **British Columbia V6E 1M2 (CA)**
- **MATSUURA, Shunji**
  **Vancouver**
  **British Columbia V6K 1A9 (CA)**

(74) Representative: **TLIP Limited**
**Murgitroyd House**
**165-169 Scotland Street**
**Glasgow G5 8PL (GB)**

(56) References cited:
**WO-A1-2005/122052**     **WO-A2-2006/026985**
**CA-A1- 2 902 015**     **US-A1- 2005 273 306**
**US-A1- 2005 273 306**     **US-A1- 2007 239 366**

- **RUBIN N. C.: "A Hybrid Classical/Quantum Approach for Large-Scale Studies of Quantum Systems with Density Matrix Embedding Theory", ARXIV.ORG, CORNELL UNIVERSITY LIBRARY, 201 OLIN LIBRARY CORNELL UNIVERSITY ITHACA, NY 14853, 21 October 2016 (2016-10-21), XP080816270**
- **HUMBLE T. S. ET AL: "Software systems for high-performance quantum computing", 2016 IEEE HIGH PERFORMANCE EXTREME COMPUTING CONFERENCE (HPEC), IEEE, 13 September 2016 (2016-09-13), pages 1 - 8, XP033012796, DOI: 10.1109/HPEC.2016.7761628**

**(Cont. next page)**

- REIHER M. ET AL: "Elucidating Reaction Mechanisms on Quantum Computers", ARXIV.ORG, CORNELL UNIVERSITY LIBRARY, 201 OLIN LIBRARY CORNELL UNIVERSITY ITHACA, NY 14853, 11 May 2016 (2016-05-11), XP080700951
- SHEN Y. ET AL: "Quantum Implementation of Unitary Coupled Cluster for Simulating Molecular Electronic Structure", ARXIV.ORG, CORNELL UNIVERSITY LIBRARY, 201 OLIN LIBRARY CORNELL UNIVERSITY ITHACA, NY 14853, 1 June 2015 (2015-06-01), XP081396619, DOI: 10.1103/PHYSREVA.95.020501
- VEIS L. ET AL: "Quantum computing applied to calculations of molecular energies: CH2 benchmark", ARXIV.ORG, CORNELL UNIVERSITY LIBRARY, 201 OLIN LIBRARY CORNELL UNIVERSITY ITHACA, NY 14853, 20 August 2010 (2010-08-20), XP080494159, DOI: 10.1063/1.3503767
- O'MALLEY P. J. J. ET AL: "Scalable Quantum Simulation of Molecular Energies", PHYSICAL REVIEW X, vol. 6, no. 3, 18 July 2016 (2016-07-18), XP055462912, DOI: 10.1103/PhysRevX.6.031007
- WENDIN G.: "Quantum information processing with superconducting circuits: a review", REPORTS ON PROGRESS IN PHYSICS, INSTITUTE OF PHYSICS PUBLISHING, BRISTOL, GB, vol. 80, no. 10, 1 September 2017 (2017-09-01), pages 106001, XP020319813, ISSN: 0034-4885, [retrieved on 20170901], DOI: 10.1088/1361-6633/AA7E1A
- KASSAL I. ET AL: "Simulating Chemistry Using Quantum Computers", ANNUAL REVIEW OF PHYSICAL CHEMISTRY., vol. 62, no. 1, 5 May 2011 (2011-05-05), US, pages 185 - 207, XP055827409, ISSN: 0066-426X, DOI: 10.1146/annurev-physchem-032210-103512

**Description**

**CROSS-REFERENCE**

This application claims the benefit of U.S. Provisional Patent Application Serial Number 62/593,060, filed November 30, 2017.

**BACKGROUND**

In chemistry and biology, the identification and the prediction of the electronic structure and the most energetically stable conformers of a molecule have significant importance as molecular function is inherently embedded in molecular conformation. For example, it is known that the reaction rate in a catalyzed reaction can vary significantly based on which of several different conformations of the catalyst are used. As another example, it is known that a protein is functional only when it forms a certain tertiary structure.

In order to accurately identify and predict the electronic structure and the most stable conformers, highly accurate quantum chemistry methods, such as Coupled-Cluster theory (CC) or Full Configuration Interaction (Full CI), are typically performed. However, the computational costs of such methods can exponentially increase with the size of a molecule, and they often become intractable in cases where the size of a molecule exceeds about 50 atoms for CC, and about 10 atoms for Full CI, even when performed on the current state-of-the-art classical computers. Therefore, a highly efficient and accurate computational framework is desirable to identify the most stable conformers of industry-relevant chemical compounds and biologically-relevant large molecules.

Quantum computing (QC) technology may be capable of computing the quantum mechanical energy and/or electronic structure of a molecule with exponentially less computational resources compared to classical computing. Thus, high-accuracy quantum chemistry calculations that are intractable using classical computing may become tractable using the QC approaches. However, QC approaches may face challenges, such as the high expense and rarity of QC resources. In addition, increasing the number of qubits in a quantum computer is a technologically challenge, which has limited the size of quantum computing devices. In addition, qubits are very sensitive to noise and environmental effects, which may cause them to decohere in a very short amount of time, thereby providing a relatively small window for running meaningful calculations.

**SUMMARY**

Recognized herein is the need for quantum algorithms and circuits that efficiently leverage current and near-term quantum computing systems to solve complex quantum chemistry problems. One such approach is to decompose a given industry-sized problem into subproblems, identify the more complex subproblems, and then use quantum computers to process only those subproblems that are challenging for classical computers.

Systems and methods provided herein utilize problem decomposition (PD) techniques in quantum chemistry toward identification and prediction of the electronic structure and a set of the most energetically stable conformers of a molecule. Such PD techniques may include the fragment molecular orbital (FMO) method, the divide-and-conquer (DC) method, the density matrix embedding theory (DMET) method, the density matrix renormalization group (DMRG) method, tensor networks, the method of increments, and others, as described herein.

In quantum chemistry, PD techniques have been developed to efficiently compute molecular energies and/or electronic structures with reasonable accuracy using classical computing. In PD techniques, the molecule may be decomposed into smaller fragments such that the quantum mechanical energy and/or electronic structure computation becomes tractable for each fragment. The quantum mechanical energy and/or electronic structure computation may then be performed individually for each fragment. The quantum mechanical energy and/or electronic structure computations resulting from each fragment may be recombined into a solution for the original molecule.

Systems and methods provided herein to perform PD techniques on a QC platform may enable quantum mechanical energy and/or electronic structure computations to be performed with a high level of accuracy for each fragment. Further, the small size of each fragment may allow highly accurate computations to be performed on QC devices on which the scale of computations is rather restricted, thereby obtaining the energies and/or electronic structures of complex, industry-relevant molecules efficiently and accurately.

The identification of the electronic structure and the most energetically stable conformers of a molecule is a fundamental process in chemistry- and biology-related research and development. While such processes may be performed by actually synthesizing the molecule and using a variety of physicochemical measurements to identify its electronic structure and conformations, such experimental processes typically require a very large amount of resources, such as human effort and time. Thus, highly efficient and accurate computational methods and systems, such as those provided by the present disclosure, may significantly reduce the need for such resources and render common R&D processes more efficient. Further, methods and systems described herein can be applied not only to single chemical systems structures (e.g., chemical compounds and biomolecules) but also to molecular aggregates with different associations. For example, methods and systems disclosed herein may be applied toward the identification of the most stable binding orientation of a drug candidate, relative to a target protein, determined from an ensemble of possible binding orientations.

The present disclosure provides methods and systems for using a hybrid architecture of quantum and classical computing processors to efficiently identify the electronic structure and the stable conformations of a chemical system (e.g., a molecule). A method may comprise obtaining an indication of a molecule; calculating or obtaining an ensemble of conformations of the molecule; and decomposing the chemical system into fragments (subsystems) for each conformation (which may be optionally stored in a list). The method may further comprise calculating the fermionic Hamiltonian (molecular Hamiltonian or electronic Hamiltonian) of each fragment of each conformation of the molecule; transforming each fermionic Hamiltonian to an equivalent qubit Hamiltonian; transforming the qubit Hamiltonian into a quantum circuit; calculating an initial state for qubits involved in the calculation of the total quantum mechanical energy and/or electronic structure; generating (e.g., through computational simulation) molecular quantum mechanical energy and/or electronic structure on a quantum hardware or classical simulator of a quantum circuit; and combining the energies and/or electronic structures for the plurality of the fragments to obtain an estimation of the total energy of the chemical system. The method may further comprise repeating these operations for all conformations in the ensemble of conformations and sorting the conformations in the ensemble of conformations based on the estimated total quantum mechanical energy and/or electronic structure. The method may further comprise providing an indication of the sorted conformation ensemble (e.g., in a list).

In one aspect, the present invention provides a method for performing a quantum mechanical energy or electronic structure calculation for a chemical system, the method being implemented by a hybrid computing system comprising a classical computer and at least one non-classical computer, the method comprising: (a) determining an ensemble of conformations of the chemical system; (b) decomposing at least one conformation within the ensemble into a plurality of molecular fragments; (c) determining, using the hybrid computing system, quantum mechanical energies or electronic structures of each of at least a subset of the plurality of molecular fragments; (d) combining the quantum mechanical energies or electronic structure determined in (c); and (e) electronically outputting a report indicative of the quantum mechanical energies or electronic structure combined in (d).

In some embodiments, the at least one non-classical computer comprises at least one quantum computer. In some embodiments, the at least one quantum computer comprises one or more members selected from the group consisting of a quantum hardware device and a classical simulator of a quantum circuit. In some embodiments, a given quantum mechanical energy of the quantum mechanical energies comprises nuclear-nuclear repulsion energy.

In some embodiments, the method further comprises providing an input to the hybrid computing system, the input comprising a set of atomic coordinates for the chemical system. In some embodiments, the method further comprises performing (b)-(d) for two or more conformations within the ensemble of conformations of the chemical system. In some embodiments, the method further comprises sorting the combined quantum mechanical energies or electronic structures of the at least the subset of the plurality of molecular fragments.

In some embodiments, (b) comprises applying one or more members selected from the group consisting of: a fragment molecular orbital (FMO) method, a divide-and-conquer (DC) method, a density matrix embedding theory (DMET) method, a density matrix renormalization group (DMRG) method, a tensor network, and a method of increments.

In some embodiments, (c) comprises: (a) determining a fermionic Hamiltonian (molecular Hamiltonian or electronic Hamiltonian) of a given molecular fragment of the at least the subset of the plurality of molecular fragments; (b) transforming the fermionic Hamiltonian into an equivalent qubit Hamiltonian; (c) transforming the qubit Hamiltonian into a quantum circuit; and (d) determining, using the quantum circuit, the quantum mechanical energy or electronic structure of the given molecular fragment. In some embodiments, the method further comprises determining the quantum mechanical energy or electronic structure using a molecular Hamiltonian. In some embodiments, the method further comprises determining the quantum mechanical energy or electronic structure using an electronic Hamiltonian. In some embodiments, transforming the fermionic Hamiltonian into an equivalent qubit Hamiltonian comprises transforming a fermionic operator of a Hamiltonian to a qubit operator.

In some embodiments, the method further comprises performing an *ab initio* molecular dynamics (AIMD) simulation of the chemical system. In some embodiments, the AIMD simulation comprises: prior to (a), obtaining an indication of a chemical system, the indication comprising coordinates of each particle of a plurality of particles in the chemical system and velocities of each particle in the chemical system; and subsequent to (d): (i) determining, from the combined energy or electronic structure, a force on each particle in the systems; (ii) updating the coordinates of each particles in the chemical system and the velocities of each particle in the chemical system; and (iii) electronically outputting a report indicative of the coordinates or velocities. In some embodiments, (i) comprises applying Jordan's quantum algorithm for numerical gradient estimation to the quantum mechanical energy or electronic structure. In some embodiments, (ii) comprises applying one or more members selected from the group consisting of: a Verlet procedure, a velocity Verlet procedure, symplectic integration, Runge-Kutta integration, and Beeman integration.

In another aspect, a system for performing a quantum mechanical energy or electronic structure calculation for a chemical system may comprise: memory comprising instructions for performing the quantum mechanical energy or electronic structure calculation for the chemical system; and a hybrid computing system operatively coupled to the memory, wherein the hybrid computing system comprises at least one classical computer and at least one non-classical computer, wherein the hybrid computing system is configured to execute the instructions to at least: (a) determine an ensemble of conformations of the chemical system; (b) decompose at least one conformation within the ensemble into a plurality of molecular fragments; (c) determine quantum mechanical energies or electronic structures of at least a subset of the plurality of molecular fragments; (d) combine the quantum mechanical energies or electronic structures determined in (c); and (e) electronically output a report indicative of the quantum mechanical energies or electronic structures combined in (d).

In another aspect, a non-transitory computer readable medium may comprise machine-executable code that upon execution by a hybrid computing system comprising at least one classical computer and at least one non-classical computer, implements a method for performing a quantum mechanical energy or electronic structure calculation for a chemical system, the method comprising: (a) determining an ensemble of conformations of the chemical system; (b) decomposing at least one conformation within the ensemble into a plurality of molecular fragments; (c) determining quantum mechanical energies or electronic structures of at least a subset of the plurality of molecular fragments; (d) combining the quantum mechanical energies or electronic structures determined in (c); and (e) electronically outputting a report indicative of the quantum mechanical energies or electronic structures combined in (d).

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

## BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**FIG. 1** shows a flowchart for an example of a method for providing an indication of a sorted list of conformers of a molecule using problem decomposition techniques on quantum computing hardware, in accordance with embodiments disclosed herein.

**FIG. 2** shows a flowchart for an example of a method for providing an indication of the quantum mechanical energy and/or electronic structure of a subsystem, which is defined by problem decomposition techniques, on quantum computing hardware, in accordance with embodiments disclosed herein.

**FIG. 3** shows a flowchart for an example of a method for providing an indication of the expectation value of the Hamiltonian, on quantum computing hardware, in accordance with embodiments disclosed herein.

**FIG. 4** is an exemplary illustration of n-heptane, where the dotted lines indicate the bond detached atom in the fragment molecular orbital (FMO) fragmentation.

**FIG. 5** is an exemplary illustration of n-heptane, showing comparisons between results obtained by exact CCSD and divide-and-conquer CCSD (DC-CCSD), and between results obtained by exact CCSD and fragment molecular orbital CCSD (FMO-CCSD).

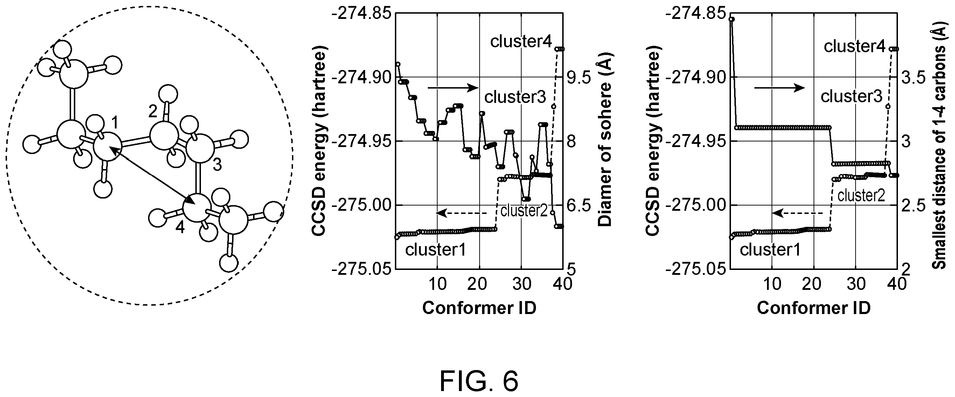

**FIG. 6** is an exemplary illustration of n-heptane, showing the minimal sphere (dotted circle) necessary to accommodate the conformer, and the distance (solid line) between the end carbon-atoms involved in a dihedral angle (1-4 distance) [left]; a plot showing the relation between the total quantum mechanical energy (left arrow) and the diameter of the minimal sphere (right arrow) for each of the conformers, which are sorted based on the total quantum mechanical energy [middle]; and a plot showing the relation between the total quantum mechanical energy (left arrow) and the smallest 1-4 distance (right arrow) for each conformer [right].

**FIG. 7** is an exemplary illustration of 3-methylheptane, where the dotted lines indicate the bond detached atoms in the fragment molecular orbital (FMO) fragmentation.

**FIG. 8** shows the quantum mechanical energy distribution for n-heptane (blue) and 3-methylheptane (red).

**FIG. 9** is an exemplary illustration of 3-methylheptane, showing comparisons between results obtained by exact CCSD and divide-and-conquer CCSD (DC-CCSD), and between results obtained by exact CCSD and fragment molecular orbital CCSD (FMO-CCSD).

**FIG. 10** shows a computer control system that is programmed or otherwise configured to implement methods provided herein.

FIG. 11 shows a flowchart for an example of a method of increments for performing problem decomposition.

FIG. 12 shows molecular orbitals, atoms, molecular fragments, and molecules used as bases for the method of increments.

FIG. 13 shows a flowchart for an example of a method for performing *ab initio* molecular dynamics (AIMD) on a molecule using problem decomposition techniques on quantum computing hardware, in accordance with embodiments disclosed herein.

FIG. 14 shows a flowchart for an example of a method **1400** for calculating the force on each particle of a system in an *ab initio* molecular dynamics (AIMD) simulation, in accordance with embodiments disclosed herein.

FIG. 15 shows examples of systems or combinations of systems that may be used to solve problems of the present disclosure, such as a quantum chemistry problem or simulation.

## DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Unless otherwise defined, all technical terms used herein have the same meaning as commonly understood by one of ordinary skill in the art to which this invention belongs. As used in this specification and the appended claims, the singular forms "a," "an," and "the" include plural references unless the context clearly dictates otherwise. Any reference to "or" herein is intended to encompass "and/or" unless otherwise stated.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

In the following detailed description, reference is made to the accompanying figures, which form a part hereof. In the figures, similar symbols typically identify similar components, unless context dictates otherwise. The illustrative embodiments described in the detailed description, figures, and claims are not meant to be limiting. Other embodiments may be utilized, and other changes may be made, without departing from the scope of the subject matter presented herein. It will be readily understood that the aspects of the present disclosure, as generally described herein, and illustrated in the figures, can be arranged, substituted, combined, separated, and designed in a wide variety of different configurations, all of which are explicitly contemplated herein.

The present invention provides methods of applying problem decomposition (PD) techniques in quantum chemistry toward identification and prediction of the quantum mechanical energy and/or electronic structure of a chemical system or to identify a set of the most energetically stable conformers of a molecule. Systems and methods provided herein to perform PD techniques on a QC platform may enable quantum mechanical energy and/or electronic structure computations to be performed with a high level of accuracy for each fragment. Further, the small size of each fragment may allow highly accurate computations to be performed on QC devices on which the scale of computations is rather restricted, thereby obtaining the energies and/or electronic structures of complex, industry-relevant molecules efficiently and accurately. Methods and systems described herein can be applied not only to single chemical systems but also to molecular aggregates with different association structures. For example, methods and systems disclosed herein may be applied toward the identification of the most stable binding orientation of a drug candidate to a target protein from the ensemble of possible binding orientations.

In some embodiments, a classical computer may be configured to perform one or more classical algorithms. A classical algorithm (or classical computational task) may comprise an algorithm (or computational task) that is able to be executed by one or more classical computers without the use of a quantum computer, a quantum-ready computing service, or a quantum-enabled computing service. A classical algorithm may comprise a non-quantum algorithm. A classical computer may comprise a computer which does not comprise a quantum computer, a quantum-ready computing service, or a quantum-enabled computer. A classical computer may process or store data represented by digital bits (e.g., zeroes ("0") and ones ("1")) rather than quantum bits (qubits). Examples of classical computers include, but are not limited to, server computers, desktop computers, laptop computers, notebook computers, sub-notebook computers, netbook computers, netpad computers, set-top computers, media streaming devices, handheld computers, Internet appliances, mobile smartphones, tablet computers, personal digital assistants, video game consoles, and vehicles.

The hybrid computing system may comprise a classical computer and quantum computer. The quantum computer may be configured to perform one or more quantum algorithms for solving a computational problem (e.g., at least a portion of a quantum chemistry simulation). The one or more quantum algorithms may be executed using a quantum computer, a quantum-ready computing service, or a quantum-enabled computing service. For instance, the one or more quantum algorithms may be executed using the systems or methods described in U.S. Patent Publication No. 2018/0107526, entitled "METHODS AND SYSETMS FOR QUANTUM READY AND QUANTUM ENABLED COMPUTATIONS". The classical computer may comprise at least one classical processor and computer memory, and may be configured to perform one or more classical algorithms for solving a computational problem (e.g., at least a portion of a quantum chemistry simulation). The digital computer may comprise at least one computer processor and computer memory, wherein the digital computer may include a computer program with instructions executable by the at least one computer processor to render an application. The application may facilitate use of the quantum computer and/or the classical computer by a user.

Some implementations may use quantum computers along with classical computers operating on bits, such as personal desktops, laptops, supercomputers, distributed computing, clusters, cloud-based computing resources, smartphones, or tablets.

The system may comprise an interface for a user. In some embodiments, the interface may comprise an application programming interface (API). The interface may provide a programmatic model that abstracts away (e.g., by hiding from the user) the internal details (e.g., architecture and operations) of the quantum computer. In some embodiments, the interface may minimize a need to update the application programs in response to changing quantum hardware. In some embodiments, the interface may remain unchanged when the quantum computer has a change in internal structure.

The present disclosure provides systems and methods that may include quantum computing or use of quantum computing. Quantum computers may be able to solve certain classes of computational tasks more efficiently than classical computers. However, quantum computation resources may be rare and expensive, and may involve a certain level of expertise to be used efficiently or effectively (e.g., cost-efficiently or cost-effectively). A number of parameters may be tuned in order for a quantum computer to deliver its potential computational power.

Quantum computers (or other types of non-classical computers) may be able to work alongside classical computers as co-processors. A hybrid architecture (e.g., computing system) comprising a classical computer and a quantum computer can be very efficient for addressing complex computational tasks, such as quantum chemistry simulations. Systems and methods disclosed herein may be able to efficiently and accurately decompose or break down a given quantum chemistry problem and delegate appropriate components of the quantum chemistry simulations to the quantum computer or the classical computer.

Although the present disclosure has made reference to quantum computers, methods and systems of the present disclosure may be employed for use with other types of computers, which may be non-classical computers. Such non-classical computers may comprise quantum computers, hybrid quantum computers, quantum-type computers, or other computers that are not classical computers. Examples of non-classical computers may include, but are not limited to, Hitachi Ising solvers, coherent Ising machines based on optical parameters, and other solvers which utilize different physical phenomena to obtain more efficiency in solving particular classes of problems.

## Classical computer

In some embodiments, the systems, media, networks, and methods described herein comprise a classical computer, or use of the same. In some embodiments, the classical computer includes one or more hardware central processing units (CPUs) that carry out the classical computer's functions. In some embodiments, the classical computer further comprises an operating system (OS) configured to perform executable instructions. In some embodiments, the classical computer is connected to a computer network. In some embodiments, the classical computer is connected to the Internet such that it accesses the World Wide Web. In some embodiments, the classical computer is connected to a cloud computing infrastructure. In some embodiments, the classical computer is connected to an intranet. In some embodiments, the classical computer is connected to a data storage device.

In accordance with the description herein, suitable classical computers may include, by way of non-limiting examples, server computers, desktop computers, laptop computers, notebook computers, sub-notebook computers, netbook computers, netpad computers, set-top computers, media streaming devices, handheld computers, Internet appliances, mobile smartphones, tablet computers, personal digital assistants, video game consoles, and vehicles. Smartphones may be suitable for use with methods and systems described herein. Select televisions, video players, and digital music players, in some cases with computer network connectivity, may be suitable for use in the systems and methods described herein. Suitable tablet computers may include those with booklet, slate, and convertible configurations.

In some embodiments, the classical computer includes an operating system configured to perform executable instructions. The operating system may be, for example, software, including programs and data, which manages the device's hardware and provides services for execution of applications. Suitable server operating systems include, by way of non-limiting examples, FreeBSD, OpenBSD, NetBSD®, Linux, Apple® Mac OS X Server®, Oracle® Solaris®, Windows Server®, and Novell® NetWare®. Suitable personal computer operating systems may include, by way of non-limiting examples, Microsoft® Windows®, Apple® Mac OS X®, UNIX®, and UNIX-like operating systems such as GNU/Linux®. In some embodiments, the operating system is provided by cloud computing. Suitable mobile smart phone operating systems may include, by way of non-limiting examples, Nokia® Symbian® OS, Apple® iOS®, Research In Motion® BlackBerry OS®, Google® Android®, Microsoft® Windows Phone® OS, Microsoft® Windows Mobile® OS, Linux®, and Palm® WebOS®. Suitable media streaming device operating systems may include, by way of non-limiting examples, Apple TV®, Roku®, Boxee®, Google TV®, Google Chromecast®, Amazon Fire®, and Samsung® HomeSync®. Suitable video game console operating systems may include, by way of non-limiting examples, Sony® PS3®, Sony® PS4®, Microsoft® Xbox 360®, Microsoft Xbox One, Nintendo® Wii®, Nintendo® Wii U®, and Ouya®.

In some embodiments, the classical computer includes a storage and/or memory device. In some embodiments, the storage and/or memory device is one or more physical apparatuses used to store data or programs on a temporary or permanent basis. In some embodiments, the device is volatile memory and requires power to maintain stored information. In some embodiments, the device is non-volatile memory and retains stored information when the classical computer is not powered. In some embodiments, the non-volatile memory comprises flash memory. In some embodiments, the non-volatile memory comprises dynamic random-access memory (DRAM). In some embodiments, the non-volatile memory comprises ferroelectric random access memory (FRAM). In some embodiments, the non-volatile memory comprises phase-change random access memory (PRAM). In other embodiments, the device is a storage device including, by way of non-limiting examples, CD-ROMs, DVDs, flash memory devices, magnetic disk drives, magnetic tapes drives, optical disk drives, and cloud computing based storage. In some embodiments, the storage and/or memory device is a combination of devices such as those disclosed herein.

In some embodiments, the classical computer includes a display to send visual information to a user. In some embodiments, the display is a cathode ray tube (CRT). In some embodiments, the display is a liquid crystal display (LCD). In some embodiments, the display is a thin film transistor liquid crystal display (TFT-LCD). In some embodiments, the display is an organic light emitting diode (OLED) display. In some embodiments, on OLED display is a passive-matrix OLED (PMOLED) or active-matrix OLED (AMOLED) display. In some embodiments, the display is a plasma display. In other embodiments, the display is a video projector. In some embodiments, the display is a combination of devices such as those disclosed herein.

In some embodiments, the classical computer includes an input device to receive information from a user. In some embodiments, the input device is a keyboard. In some embodiments, the input device is a pointing device including, by way of non-limiting examples, a mouse, trackball, track pad, joystick, game controller, or stylus. In some embodiments, the input device is a touch screen or a multi-touch screen. In some embodiments, the input device is a microphone to capture voice or other sound input. In some embodiments, the input device is a video camera or other sensor to capture motion or visual input. In some embodiments, the input device is a Kinect, Leap Motion, or the like. In some embodiments, the input device is a combination of devices such as those disclosed herein.

## Non-transitory computer readable storage medium

In some embodiments, the systems and methods described herein include one or more non-transitory computer readable storage media encoded with a program including instructions executable by the operating system of an optionally networked digital processing device. In some embodiments, a computer readable storage medium is a tangible component of a classical computer. In some embodiments, a computer readable storage medium is optionally removable from a classical computer. In some embodiments, a computer readable storage medium includes, by way of non-limiting examples, CD-ROMs, DVDs, flash memory devices, solid state memory, magnetic disk drives, magnetic tape drives, optical disk drives, cloud computing systems and services, and the like. In some cases, the program and instructions are permanently, substantially permanently, semi-permanently, or non-transitorily encoded on the media.

Embodiments of the disclosed method for efficiently identifying the stable conformations of a chemical system are described below.

## Identification of a Target Chemical System

A hybrid computing system comprising a classical computer and a quantum computer may be used to perform a quantum mechanical energy and/or electronic structure calculation for a chemical system. For example, such a hybrid computing system may be used to perform a method for efficiently identifying the stable conformations of a chemical system (e.g., a molecule).

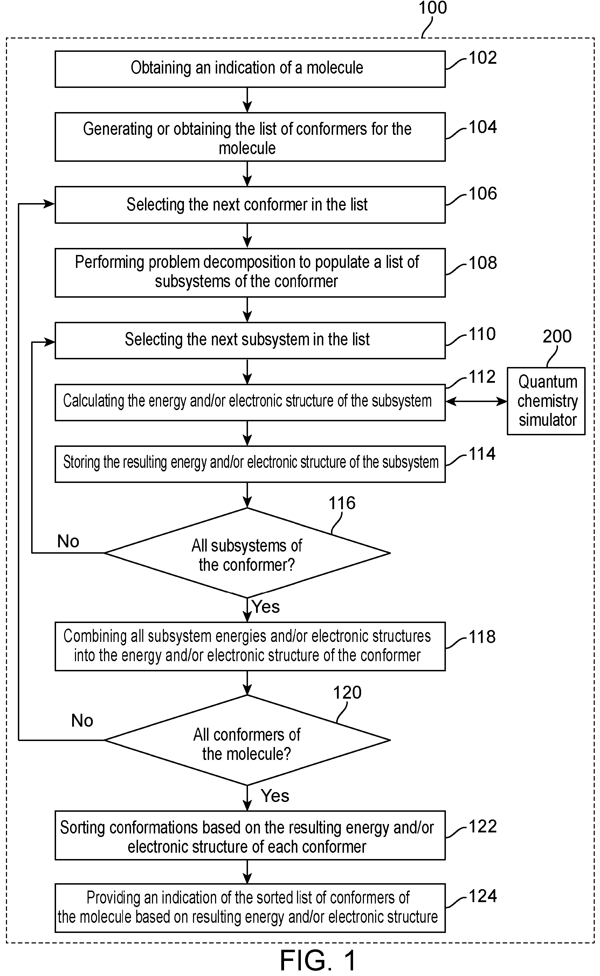

**FIG. 1** shows a flowchart for an example of a method **100** for providing an indication of a sorted list of conformers of a molecule using problem decomposition techniques on quantum computing hardware.

The method **100** may comprise obtaining an indication of an input molecule according to **operation 102.** The method **100** disclosed herein may be applicable to any type of chemical system. The chemical system may comprise, for example, an organic compound, an inorganic compound, a polymer, a peptide, a polypeptide, a protein, a nucleic acid, a carbohydrate, etc. Methods disclosed herein may also be applicable to complexes of molecules, such as one or more protein-drug complex (including or excluding solvent molecules).

## Generation of an Ensemble of Conformers

The method **100** may comprise determining an ensemble of conformations of the chemical system. For example, the method **100** may comprise generating an ensemble (e.g., list) of conformers for the input chemical system according to operation **104.** A variety of different approaches may be used to enumerate conformers for a given chemical system. In some embodiments, an exhaustive conformation sampler can be used, in which the conformation of the molecule is sampled by varying all the dihedral angles around the rotatable bonds in the chemical system. In some embodiments, Monte Carlo simulation or molecular dynamics simulation may be performed to generate the ensemble of conformers. In another embodiment, the ensemble of conformers for the molecule is given as an input together with the chemical system information.

## Selection and Processing of Conformers from the Ensemble of Conformers

The method **100** may comprise, according to operation **106,** selecting a conformer from the ensemble or list (e.g., ordered list) of conformers, and performing any at least 1, 2, 3, 4, 5, 6, or 7, or at most any 7, 6, 5, 4, 3, 2, or 1 of operations **108, 110, 112, 114, 116, 118,** and/or **120** for the conformed select. Any at least 1, 2, 3, 4, 5, 6, or 7, or at most any 7, 6, 5, 4, 3, 2, or 1 of operations **108, 110, 112, 114, 116, 118,** and/or **120** may be performed for each conformer in the ensemble of conformers.

## *(a) PD Fragmentation of a Chemical System*

The method **100** may comprise decomposing at least one conformation within the ensemble into a plurality of molecular fragments. For example, the method **100** may comprise decomposing (e.g., performing problem decomposition on) the chemical system into a plurality (e.g., a list) of smaller fragments or subsystems according to operation **108.** The specific scheme for decomposing the system into subsystems may vary depending on the PD technique used. Generally, a suitable PD fragment ("fragment") size may be selected such that the computational resources required to process the fragment do not exceed the capability of the quantum-classical hardware to be used.

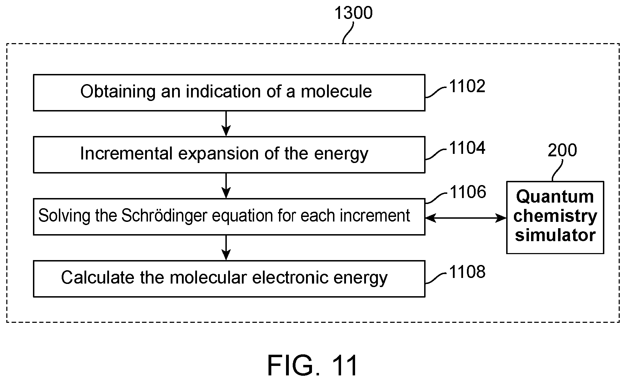

Various fragmentation approaches for chemical systems may be suitable for use, including but not limited to: (i) Divide and Conquer (DC), (ii) Fragment Molecular Orbitals (FMO), (iii) Density Matrix Embedding Theory (DMET), (iv) Density Matrix Renormalization Group (DMRG), (v) Tensor Networks, (vi) the method of increments (as described herein with respect to **FIG. 11),** and others.

For example, the FMO method was first described by [Kitaura et al., "Fragment molecular orbital method: an approximate computational method for large molecules," Chemical Physics Letters, 1999, 313, 701]. The FMO method has been applied to many systems, such as those described by [Fedorov et al., "Exploring chemistry with the fragment molecular orbital method," Physical Chemistry Chemical Physics, 2012, 14, 7562.

For example, the DC method was first described by [Yang, "Direct calculation of electron density in density-functional theory: Implementation for benzene and a tetrapeptide," Physical Review A, 1991, 44, 7823]. The DC method has been further developed and described, for example, by [Akama et al., "Implementation of divide-and-conquer method including Hartree-Fock exchange interaction," Journal of Computational Chemistry, 2007, 28, 2003] and [Kobayashi et al., "Divide-and-conquer approaches to quantum chemistry: Theory and implementation," in Linear-Scaling Techniques in Computational Chemistry and Physics: Methods and Applications, edited by Zalesny et al. (Springer Netherlands, Dordrecht, 2011), 97-127].

For example, DMET was first described by [Knizia et al., "Density Matrix Embedding: A Simple Alternative to Dynamical Mean-Field Theory," Physics Review Letters, 2012, 109, 186404]. DMET was further developed and described, for example, by [Wouters et al., "A Practical Guide to Density Matrix Embedding Theory in Quantum Chemistry," Journal of Chemical Theory and Computation, 2016, 12, 2706].

FIG. 11 shows a flowchart for an example of a method **1100** of increments for performing problem decomposition. The method **1100** may be referred to as a "method of increments" in general, or may be variously referred depending on the quantum chemistry method utilized. For example, the utilization of unitary coupled cluster (UCC) method in the method of increments may be referred to as "incremental unitary coupled-cluster" (iUCC) method. The method **1100** may comprise obtaining an indication of a molecule according to operation **1102.** The method **1100** disclosed herein may be applicable to any type of molecule. The molecule may comprise, for example, an organic compound, an inorganic compound, a polymer, a peptide, a polypeptide, a protein, a nucleic acid, a carbohydrate, etc. Methods disclosed herein may also be applicable to complexes of molecules, such as one or more protein-drug complex (including or excluding solvent molecules).

The method **1100** may comprise performing an incremental expansion of the energy of the molecule, according to operation **1104.** The incremental expansion may be performed according to Equation (1):

$$E_C = \sum_i \epsilon_i + \sum_{i>j} \epsilon_{ij} + \sum_{i>j>k} \epsilon_{ijk} \qquad (1)$$

Here, the correlation energy $E_C$ (the difference between the total molecular energy and the mean-field Hartree-Fock energy) is expressed as an n-body Bethe-Goldstone expansion. The individual n-body correlation energy contributions are defined by Equation (2):

$$\epsilon_i = E_C(i) \qquad (2)$$

$$\epsilon_{ij} = E_C(ij) - \epsilon_i - \epsilon_j$$

$$\epsilon_{ijk} = E_C(ijk) - \epsilon_{ij} - \epsilon_{ik} - \epsilon_{jk} - \epsilon_i - \epsilon_j - \epsilon_k$$

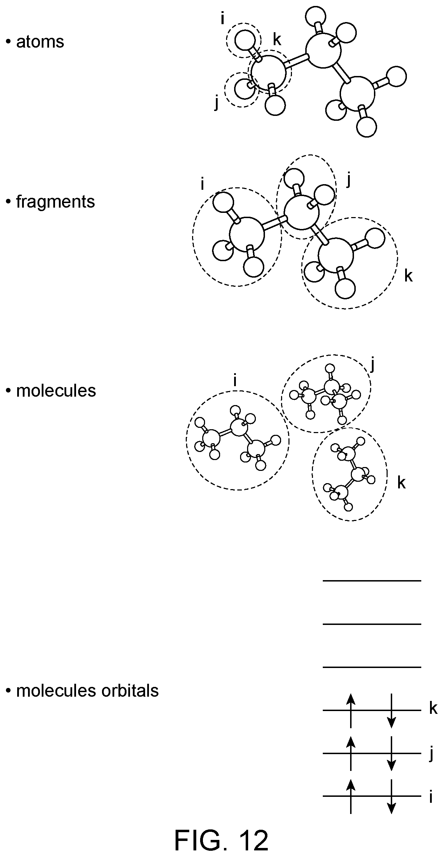

Here, $E_C(i)$ are the individual 1-body correlation energies, $E_C(ii)$ are the individual 2-body correlation energies, and $E_C(ijk)$ are the individual 3-body correlation energies. The indices $i, j$, and k may correspond to any number of molecular orbitals, atoms, molecular fragments, or whole molecules. The indices $i, j$, and $k$ may correspond to any possible combination of molecular orbitals, atoms, molecular fragments, and whole molecules. Thus, the incremental expansion may be expressed in terms of any possible combination of molecular orbitals, atoms, molecular fragments, and whole molecules. **FIG. 12** depicts molecular orbitals, atoms, molecular fragments, and molecules used as bases for the method of increments. When the incremental expansion is expressed in terms of any possible combination of atoms, fragments, and molecules, the resulting framework may become the framework of an FMO method. When the incremental expansion is expressed in terms of molecular orbitals, the resulting framework may become the framework of an incremental full configuration interaction (iFCI) method, such as that described by [Zimmerman et al., "Strong Correlation in Incremental Full Configuration Interaction," Journal of Chemical Physics, 2017, 146, 224104].

Returning to the description of **FIG. 11,** the method **1100** may further comprise solving the Schrödinger equation (e.g., by using a quantum chemistry simulator to solve a quantum chemistry problem according to method **200)** for each increment described with reference to operation **1104,** according to operation **1106.** In some embodiments, the solution of the Schrödinger equation may be achieved using a phase estimation procedure. For example, a phase estimation algorithm is described by [Aspuru-Guzik et al., "Simulated Quantum Computation of Molecular Energies," Science, 2005, 309, 1704]. In some embodiments, the solution of the Schrödinger equation may be achieved using an adiabatic quantum simulation. In some embodiments, the solution of the Schrödinger equation may be achieved by solving a unitary coupled-cluster (UCC) problem within a variational quantum eigensolver (VQE). For example, a VQE is described by [McClean et al., "The theory of variational hybrid quantum-classical algorithms," New Journal of Physics, 2016, 18, 023023]. In some embodiments, the UCC ansatz may comprise all possible excitations for a given increment. In such cases, the UCC ansatz may be equivalent to an exact solution of the Schrödinger equation or to the full configuration interaction (FCI) for each increment. In some embodiments, truncations of the UCC ansatz to lower order excitations may be used to approximate the exact results (to any possible approximation) for each increment. The solution of the Schrödinger equation may be repeated for one or more increments. For instance, the solution of the Schrödinger equation may be repeated for any possible subset of all increments or may be repeated for all increments. In some embodiments, the solution of the Schrödinger equation for each increment may be parallelized. For instance, the solution of the Schrödinger equation for each increment may be parallelized using high-performing computing architectures.

The method **1100** may further comprise calculating the quantum mechanical molecular electronic energy, according to operation **1108.** The quantum mechanical molecular electronic energy may be calculating by summing each of the incremental contributions according to Equation (1) to yield the quantum mechanical molecular correlation energy and thus the total quantum mechanical energy of the system under study.

Returning to the description of **FIG. 1,** in some embodiments, the same fragmentation scheme may be used for all conformers in the ensemble. Such a fragmentation scheme may be appropriate, for example, when the capability of the hardware is limited and the fragment size has to be very small. This fragmentation scheme may related to the error cancellation described in the Examples herein. In some embodiments, a different fragmentation scheme may be used for one or more conformers in the ensemble.

*(b) Calculation of Quantum Mechanical Energies and/or Electronic Structures for each PD Fragment*

The method **100** may comprise determining, using the hybrid computing system, quantum mechanical energies and/or electronic structures of each of at least a subset of the plurality of molecular fragments. For example, the method **100** may comprise calculating quantum mechanical energies and/or electronic structures of one or more subsystems according to operations **110, 112,** and **114).**

According to operation **110,** the next fragment or subsystem in the list may be selected. Then, the operations **112** and **114** may be considered for each PD fragment. For example, according to operation **112,** the quantum mechanical energy and/or electronic structure of the subsystem may be calculated (e.g., by using a quantum chemistry simulator to solve a quantum chemistry problem according to method **200).** According to operation **114,** the resulting quantum mechanical energy and/or electronic structure of the subsystem may be stored.

### (i) PD Molecular Hamiltonian Construction

In some embodiments, using the hybrid computing system to determine quantum mechanical energies and/or electronic structures of the each of the molecular fragments may comprise determining quantum mechanical energies and/or electronic structures of the molecular fragments (e.g., by constructing a molecular Hamiltonian or an electronic Hamiltonian), transforming the quantum mechanical energies and/or electronic structures into equivalent qubit energies and/or electronic structures (e.g., by transforming a fermionic operator of a Hamiltonian to a qubit operator), and determining, using the quantum circuit, the quantum mechanical energy and/or electronic structure of the given molecular fragment.

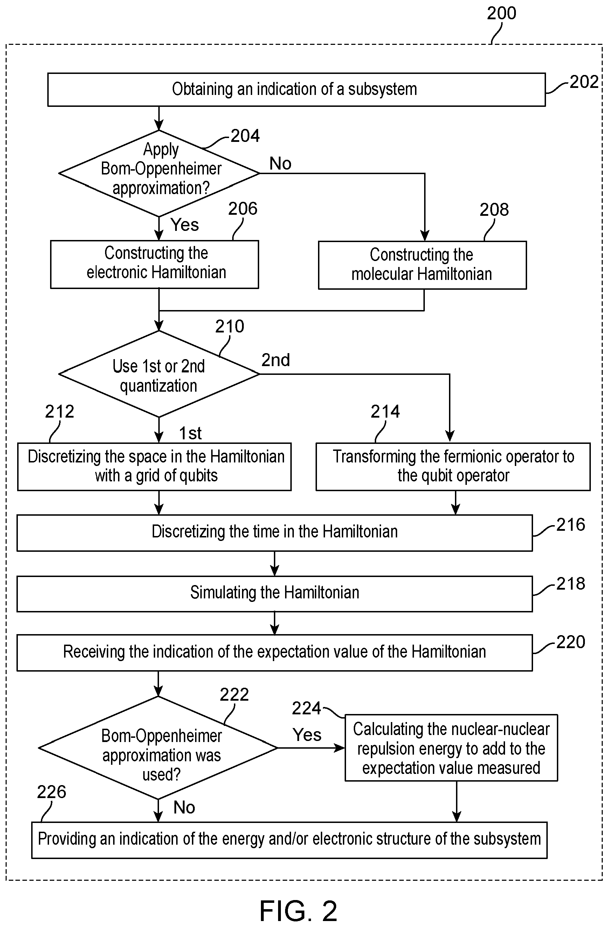

**FIG. 2** shows a flowchart for an example of a method **200** for providing an indication of the quantum mechanical energy and/or electronic structure of a subsystem, which is defined by problem decomposition techniques, on quantum computing hardware.

The method **200** may comprise obtaining an indication of a subsystem according to operation **202.** An approach to solving quantum chemistry problems using classical computing may be to use the Born-Oppenheimer approximation, in which the electron wave function and the nuclear wave function are decoupled and only the electronic Hamiltonian is solved. However, the methods disclosed herein may or may not use the Born-Oppenheimer approximation. Such an option to use or not use the Born-Oppenheimer approximation may be selected according to operation **204,** for example, by input from a user of the system.

If the Born-Oppenheimer approximation is selected by the user, then the electronic Hamiltonian for the fragment may be constructed according to operation **206.** If the Born-Oppenheimer approximation is not selected by the user, then the molecular Hamiltonian for the fragment may be constructed according to operation **208.**

The qubit Hamiltonian may be constructed for each fragment using either (a) the first quantization formalism according to operation **212,** in which the space in the Hamiltonian is discretized with a grid of qubits, or (b) the second quantization formalism according to operation **214,** in which the fermionic operator is transformed to the qubit operator. Such an option to use either the first quantization formalism or the second quantization formalism to generate the qubit Hamiltonian may be selected according to operation **210,** for example, by input from a user of the system.

For example, in the case of second quantization formalism with the Born-Oppenheimer approximation, according to **operation 206,** the electronic Hamiltonian $H^{el}$ may be written as:

$$H^{el} = \sum_{pq} h_{pq} \hat{a}_p^\dagger \hat{a}_a + \frac{1}{2} \sum_{pqrs} V_{pqrs} \hat{a}_p^\dagger \hat{a}_q^\dagger \hat{a}_r \hat{a}_s \qquad (3)$$

where $h_{pq}$ and $V_{pqrs}$ are integrals which can be efficiently precomputed on a classical computer and $\hat{a}^\dagger$ and $\hat{a}$ are creation and annihilation operators on the basis of spin orbitals. The two-operator terms in the first summation in Equation (3) may correspond to single-electron terms, and the four-operator terms in the second summation in Equation (3) may correspond to electron-electron interaction terms.

The exact form of the molecular Hamiltonian may vary depending on the PD technique as well as the framework being used, such as Full configuration interaction (Full CI) or Coupled-Cluster theory (CC).

### (ii) PD Qubit Hamiltonian Construction

According to operation **212,** when the first quantization formalism is selected, the qubit Hamiltonian may be obtained by discretizing the 3-dimensional real space into a 3-dimensional grid of qubits. Each grid point may then be represented by a qubit variable.

According to operation **214,** when the second quantization formalism is selected, the molecular Hamiltonian (which may be based on spin operators) may be transformed to a qubit Hamiltonian. The qubit Hamiltonian may be based on Pauli operators such as $\{\sigma^{\times}, \sigma^{y}, \sigma^{z}\}$ on qubits.

The spin-to-qubit Hamiltonian transformation may be accomplished in a variety of ways, including but not limited to the Jordan-Wigner transformation or the Bravyi-Kitaev transformation.

For example, the Jordan-Wigner transformation provides the following qubit Hamiltonian:

$$H^{el} = \sum_{pqrs} \sum_{abcd} g_{pqrs}^{abcd} \otimes_{p>i>q} \sigma_i^z \otimes_{r>j>s} \sigma_j^z (\sigma_p^a \sigma_q^b \sigma_r^c \sigma_s^d) \tag{4}$$

Here, $\otimes$ indicates an outer product and $g_{pqrs}^{abcd}$ is the constant originated from $h_{pq}$ and $V_{pqrs}$ in Equation (3). The set of indices $\{p, q, r, s\}$ may be summed over the spin orbitals. The set of indices $\{a, b, c, d\}$ may be either $x$ or $y$.

According to operation 216, the time in the Hamiltonian may be discretized, in preparation for performing the simulation of the Hamiltonian.

### (iii) Circuit Preparation

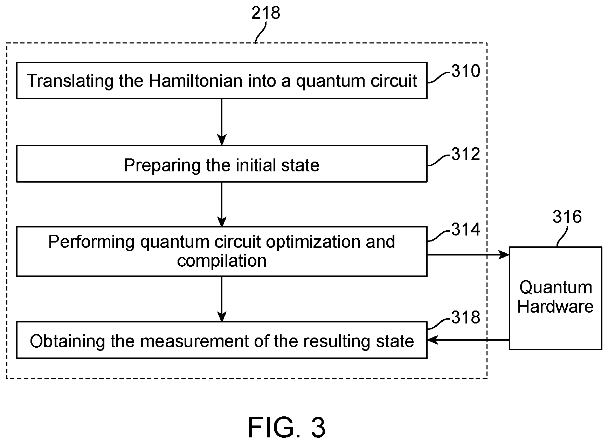

According to operation 218, the qubit Hamiltonian may be simulated. The qubit Hamiltonian may be simulated by performing any at least 1, 2, 3, or 4, or at most 4, 3, 2, or 1 of operations 310, 312, 314, and 318 disclosed herein with respect to FIG. 3.

A bottleneck in the process of accurately differentiating the molecular conformations may be performing the total quantum mechanical energy and/or electronic structure calculation. To help ease this bottleneck, PD techniques may be used to break up a problem into smaller, more manageable pieces. In some embodiments, the total quantum mechanical energy and/or electronic structure calculation for each of a subset of sub-problems can be performed using a quantum computer. In some embodiments, the quantum computation process for each of a subset of sub-problems can be simulated on a classical computer. The process of calculating the total quantum mechanical energy and/or electronic structure using a quantum computer may comprise running a quantum algorithm to calculate the lowest eigenvalue of a Hamiltonian describing the subproblem.

FIG. 3 shows a flowchart for an example of a method 218 for providing an indication of the expectation value of the Hamiltonian, on quantum computing hardware. The method 218 may comprise operation 218 of method 200.

The method 218 may comprise translating the Hamiltonian into a quantum circuit that matches to the characteristics of the computing system (e.g., quantum computing system or hardware, or quantum-classical system or hardware) being used (e.g., the connectivity of qubits and which gates are possible to apply) according to operation 310. Techniques for calculating the lowest energy eigenvalue of a Hamiltonian may include the phase estimation algorithm and the variational quantum eigensolver (VQE). For example, the phase estimation algorithm is described by [Aspuru-Guzik et al., "Simulated Quantum Computation of Molecular Energies," Science, 2005, 309, 1704]. For example, the VQE is described by [McClean et al., "The theory of variational hybrid quantum-classical algorithms," New Journal of Physics, 2016, 18, 023023]. These algorithms may be performed to encode the qubit Hamiltonian of a molecule or sub-molecule into the parameters of a quantum circuit.

### (iv) Initial State Preparation for Each PD Fragment

The method 218 may comprise preparing an initial state (or initial guess) for the quantum chemistry simulation on the quantum-classical hardware according to operation 312. A suitable initial state may be the Hartree Fock wavefunction. A suitable initial state may be wavefunctions obtained by post Hartree Fock methods. A suitable initial state may be prepared, for instance, using any of the systems or methods described in [Matsuura et al., "VanQver: The Variational and Adiabatically Navigated Quantum Eigensolver," arXiv:1810.11511 , October 31, 2018].

### (v) Simulation of PD Hamiltonian on Quantum-Classical Hardware

Given the quantum circuit (from operation 310) and initial state (from operation 312), method 218 may comprise simulating the qubit Hamiltonian. The method 218 may comprise compiling and executing (e.g., optimizing) the initial state and/or the qubit Hamiltonian on the quantum computer according to operation 314. In some embodiments, the quantum computer comprises a quantum hardware device 316 or a classical simulator of a quantum circuit (e.g., a quantum hardware simulator 316). For example, according to operation 314, the transformed quantum circuit and the initial qubit state may be sent to the quantum hardware device 316 or to the quantum hardware simulator 316 in order to perform the quantum chemistry simulation for each fragment.

The sending of the circuit and initial states (according to operations 310 and 312, respectively) for calculating the total quantum mechanical energy and/or electronic structure of a fragment can be done in sequence as the transformed fragments are ready, or they can all be calculated and then sent to one or more quantum hardware devices or classical simulators in parallel.

In some embodiments, a quantum computer may comprise one or more adiabatic quantum computers, quantum gate arrays, one-way quantum computers, topological quantum computers, quantum Turing machines, superconductor-based quantum computers, trapped ion quantum computers, trapped atom quantum computers, optical lattices, quantum dot computers, spin-based quantum computers, spatial-based quantum computers, Loss-DiVincenzo quantum computers, nuclear magnetic resonance (NMR) based quantum computers, solution-state NMR quantum computers, solid-state NMR quantum computers, solid-state NMR Kane quantum computers, electrons-on-helium quantum computers, cavity-quantum-electrodynamics based quantum computers, molecular magnet quantum computers, fullerene-based quantum computers, linear optical quantum computers, diamond-based quantum computers, nitrogen vacancy (NV) diamond-based quantum computers, Bose-Einstein condensate-based quantum computers, transistor-based quantum computers, and rare-earth-metal-ion-doped inorganic crystal based quantum computers. A quantum computer may comprise one or more of: quantum annealers, Ising solvers, optical parametric oscillators (OPO), and gate models of quantum computing.

In some embodiments, a classical simulator of the quantum circuit can be used which can run on a classical computer like a MacBook Pro laptop, a Windows laptop, or a Linux laptop. In some embodiments, the classical simulator can run on a cloud computing platform having access to multiple computing nodes in a parallel or distributed manner. In some embodiments, the total quantum mechanical energy and/or electronic structure calculation for a subset of fragments can be performed using the classical simulator and the total quantum mechanical energy and/or electronic structure calculation for the remainder of the fragments can be performed using the quantum hardware.

### (vi) Measurement of the resulting state

The method **218** may comprise measuring the quantum bits to provide a classical indication of the lowest eigenvalue according to operation **318**. Depending on the algorithm used, the parameters required to produce the electronic structure configuration that produced that lowest energy eigenvalue may also be provided. The basis of a measurement may be indicated by the Hamiltonian and the quantum algorithm being used. A measurement on the quantum data stored in quantum bits may transform that information into classical bits of information. In order to provide an accurate estimation of the data being measured, at least a portion of operation **218** may be repeated. In this case, the plurality of results obtained from a plurality of repeated executions of operation **218** may be averaged. Depending on the algorithm used, the parameters required to produce the electronic structure configuration that produced that lowest energy eigenvalue may also be provided.

Returning to the discussion of **FIG. 2,** after operation **218** has been performed one or more times, the method **200** may comprise measuring an indication of the expectation value of the Hamiltonian according to operation **220.**

The method **200** may comprise determining whether the Born-Oppenheimer approximation was used (e.g., in operation **204)** according to operation **222**. If the Born-Oppenheimer approximation was used, the method **200** may comprise calculating the nuclear-nuclear repulsion energy and then adding the calculated nuclear-nuclear repulsion energy to the measured expectation value, according to operation **224**. The method **200** may comprise providing an indication of the quantum mechanical energy and/or electronic structure of the subsystem according to operation **226,** thereby concluding the quantum chemistry simulation performed by the method **200.**

Returning to the discussion of **FIG. 1,** the method **100** may comprise storing the resulting quantum mechanical energy and/or electronic structure of the subsystem, such as in a list of quantum mechanical subsystem energies and/or electronic structures according to operation **112**. The method **100** may comprise determining if all subsystems of the conformer have been processed to calculate their quantum mechanical energies and/or electronic structures according to operation **100;** if not, then the next subsystem on the list may be selected (according to **operation 110)** and **operations 112** and **114** may be performed thereon.

### (c) Combining Quantum Mechanical Energies and/or Electronic Structures for PD Fragments

After one or more molecular fragments of the chemical system have been processed to calculate their quantum mechanical energies and/or electronic structures, the method for using the hybrid computing system to perform a quantum mechanical energy and/or electronic structure calculation for a chemical system may comprise combining the quantum mechanical energies and/or electronic structures determined for the molecular fragments. For example, the method for efficiently identifying the stable conformations of the chemical system may comprise recombining the energies and/or electronic structures obtained for each fragment to obtain the total quantum mechanical energy and/or electronic structure of the conformer of the whole chemical system (e.g., molecule), according to **operation 118.** The approach in **operation 118** to perform recombination of the energies and/or electronic structures of the fragments to obtain the total quantum mechanical energy and/or electronic structure of the conformer of the chemical system may be dependent on and fully described by the problem decomposition (PD) method used in **operation 108.** The resulting quantum mechanical energy and/or electronic structure of the conformer may then be stored, such as in a list of quantum mechanical conformer energies and/or electronic structures.

According to **operation 120,** it is determined if all conformers of interest of the chemical system (e.g., molecule) have been processed to calculate their quantum mechanical energies and/or electronic structures; if not, then the next conformer on the list is selected (according to **operation 106)** and **operations 108, 110, 112, 114, 116,** and **118** are performed thereon.

### Prediction of the Most Stable Conformer

After conformers of interest within the ensemble of conformers have been processed to calculate their quantum mechanical energies and/or electronic structures, the conformers provided in the ensemble of conformers can be sorted in any order, such as sorted by increasing or decreasing order of stability, according to **operation 122,** based on the estimation of total quantum mechanical energy and/or electronic structure of each of the conformers provided by the **operation 118.** Finally, according to **operation 124,** an indication of the sorted list of conformers of the chemical system is provided based on the resulting quantum mechanical energy and/or electronic structure, which provides a prediction of the most stable conformer among the ensemble of conformers of the chemical system.

The PD approach may generally provide accurate results, as indicated by studies such as those described by [Fedorov et al., "Exploring chemistry with the fragment molecular orbital method," Physical Chemistry Chemical Physics, 2012, 14, 7562]; [Kobayashi et al., "Divide-and-conquer approaches to quantum chemistry: Theory and implementation," in Linear-Scaling Techniques in Computational Chemistry and Physics: Methods and Applications, edited by Zalesny et al. (Springer Netherlands, Dordrecht, 2011), 97-127]; and [Wouters et al., "A Practical Guide to Density Matrix Embedding Theory in Quantum Chemistry," Journal of Chemical Theory and Computation, 2016, 12, 2706].

In addition, the examples below illustrate a good correlation between the energies obtained by a certain method (for example, by CCSD) with and without PD, even when the fragment size is very small. Therefore, the most stable conformer of a chemical system can either be directly identified based on the energies obtained by PD or by using the methods disclosed above to narrow down the size of the conformer ensemble for more accurate computations.

### *Ab Initio* Molecular Dynamics

The systems and methods of the present disclosure may be used to simulate evolution of molecular structures over time using *ab initio* molecular dynamics (AIMD) techniques. In such simulations, the quantum-enabled problem decomposition (PD) techniques described herein to calculate the quantum mechanical energy and/or electronic structure of a molecule (for instance, as described herein with respect to **FIG. 1, FIG. 2,** or **FIG. 3).** The quantum mechanical energy and/or electronic structure calculation may serve as the basis for a force calculation in the AIMD framework. The force on particles within the molecule (such as one or more atoms within the molecule) may be determined based on the quantum mechanical energy obtained by the quantum-enabled PD techniques described herein. The positions and velocities of the particles may then be updated using AIMD techniques.

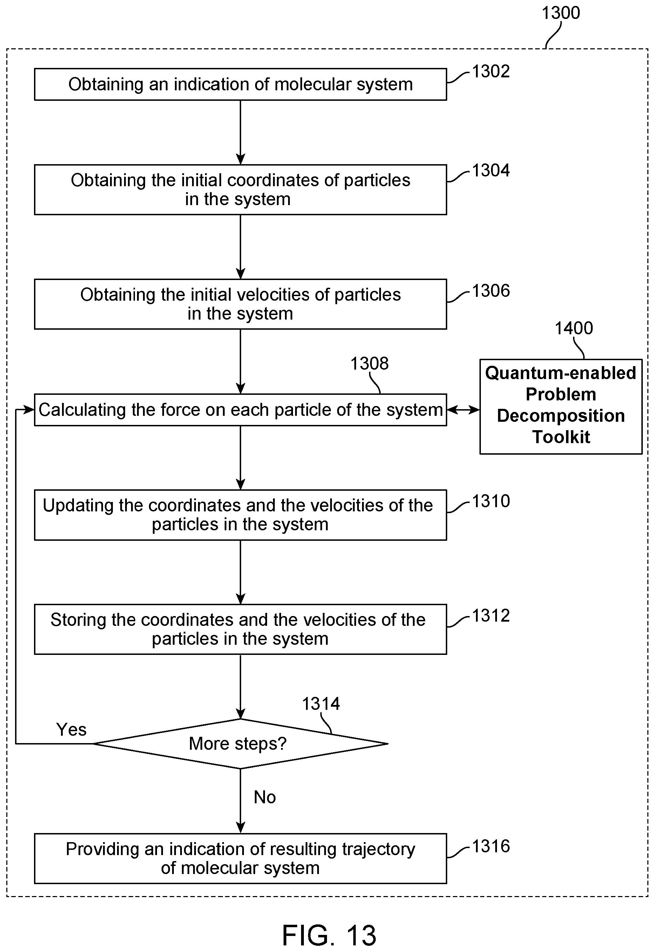

**FIG. 13** shows a flowchart for an example of a method **1300** for performing *ab initio* molecular dynamics (AIMD) on a molecule using problem decomposition techniques on quantum computing hardware.

The method **1300** may comprise obtaining an indication of an input molecule according to operation **1302.** The method **1300** disclosed herein may be applicable to any type of chemical system. The chemical system may comprise, for example, an organic compound, an inorganic compound, a polymer, a peptide, a polypeptide, a protein, a nucleic acid, a carbohydrate, etc. Methods disclosed herein may also be applicable to complexes of molecules, such as one or more protein-drug complex (including or excluding solvent molecules).

The method **1300** may comprise obtaining the initial coordinates of particles in the system according to operation **1304.** The initial coordinates of particles in the system may correspond, for instance, to the coordinates of atomic nuclei within a molecule. The initial coordinates of particles in the system may be theoretically-derived or experimentally-derived. For instance, the initial coordinates of particles in the system may be derived from a predicted molecular structure. The initial coordinates of particles in the system may be derived from experimental procedures such as X-ray crystallography, transmission electron microscopy (TEM), scanning electron microscopy (SEM), scanning tunneling electron microscopy (STEM), atomic force microscopy (AFM), solution-state nuclear magnetic resonance (NMR), solid-state NMR, or other experimental procedures. The initial coordinates of particles in the system may be obtained from a database, such as PubChem, Chemical Entities of Biological Interest (ChEBI), DrugBank, small molecule pathway database (SMPDB), ChemDB, Protein Data Bank (PDB), or other databases.

The method **1300** may comprise obtaining the initial velocities of particles in the system according to operation **1306.** The initial velocities of the particles may be obtained in a variety of manners. For instance, the initial velocities of the particles may be obtained by randomly choosing a velocity for each particle from a Maxwell-Boltzmann distribution at a desired temperature. In some cases, such a procedure may result in a net momentum of the system, resulting in an initial linear motion of the system as a whole. If desired, the initial linear motion may be removed by calculating the net momentum of the system and adjusting the initial velocity of each particle to reduce the net momentum to zero. Similarly, the procedure may result in a net angular momentum of the system, resulting in an initial rotational motion of the system as a whole. If desired, the initial rotational motion may be removed by calculating the net angular momentum of the system and adjusting the initial angular velocity of each particle to reduce the net angular momentum to zero.

During either operation **1304** or **1306,** additional parameters may be set up. For instance, a target number of molecular dynamics time steps, a time increment, a target temperature, and/or a target pressure may be specified.

The method **1300** may comprise calculating the force on each particle of the system according to operation **1308.**

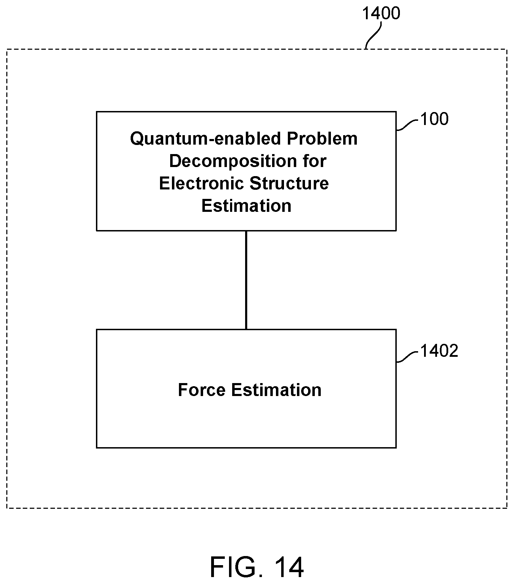

**FIG. 14** shows a flowchart for an example of a method **1400** for calculating the force on each particle of a system in an *ab initio* molecular dynamics (AIMD) simulation. The method may comprise implementing a method for quantum-enabled PD for quantum mechanical energy and/or electronic structure calculation of the system, such as method **100** described herein.

The method may further comprise estimating the force on each particle of the system according to operation **1402.** The force on each particle of the system may be calculated from the quantum mechanical energy and/or electronic structure calculation of the system. The force on each particle of the system may be calculated by a variety of procedures. For instance, the force on each particle of the system may be calculated using Jordan's quantum algorithm for numerical gradient estimation as disclosed in [Jordan, "Fast Quantum Algorithm for Numerical Gradient Estimation", Physical Review Letters, 2015, 95, 050501]. Jordan's quantum algorithm for numerical gradient estimation may be performed using quantum hardware (such as a quantum computer described herein) or on a quantum simulator (such as a quantum simulator described herein). The force on each particle of the system may be calculated using numerical gradient estimation techniques on classical hardware (such as a classical computer described herein).

Returning to the discussion of **FIG. 13,** the method **1300** may further comprise updating the coordinates and/or the velocities of the particles in the system for the next time step according to operation **1310.** The coordinates and/or the velocities of the particles in the system may be updated according to a variety of procedures. For instance, the coordinates and/or the velocities of the particles in the system may be updated using a Verlet procedure, in which the coordinates and/or velocities are updated using a series expansion of the coordinates and/or velocities based on the most recent and second-most recent time steps. The coordinates and/or velocities of the particles in the system may be updated using a velocity Verlet procedure, in which the positions are updated based on the most recent velocities, the velocities are partially updated based on the most recent forces, updated forces are calculated using the updated positions, and the velocities are fully updated based on the most recent forces. The coordinates and/or velocities of the particles in the system may be updated by numerical integration of the forces using a variety of integration techniques, such as symplectic integration, Verlet-Stoermer integration, Runge-Kutta integration, Beeman integration, or other integration techniques. During the updating of the coordinates and/or velocities of the particles in the system, a variety of thermostats and/or barostats may be applied to maintain control of the temperature and/or pressure of the system. For instance, a Langevin thermostat and an Anderson barostat may be applied.

The method **1300** may comprise storing the coordinates and/or velocities of the particles in the system, such as in a list of coordinates and/or velocities according to operation **1312.** The list of coordinates and/or velocities may comprise a trajectory of the system.

The method **1300** may comprise examining the number of molecular dynamics time steps according to operation **1314.** If the number of time steps is less than a desired number of time steps, any one or more of operations **1302, 1304, 1306, 1308, 1310,** and **1312** may be repeated until the number of time steps equals the desired number of time steps. At such a point, the method **1300** may be halted.

The method **1300** may comprise providing an indication of the resulting trajectory of the system.

## EXAMPLES

### Example 1 (n-Heptane)

The correlation between the results of total quantum mechanical energy calculations with and without PD was investigated for different conformations of a compound. The simulation results for a fixed conformation with PD may not be within chemical accuracy. However, if this is due to systematic error, then comparing two erroneous results for different conformers of the same molecule can cancel this error out and may provide an accurate relative quantum mechanical energy difference between the two conformations of the molecule. Therefore, this approach can be used to accurately pick the best conformers (e.g., the most stable conformers) based on their total quantum mechanical energy values, even without having an optimally accurate estimation of total quantum mechanical energy for each individual conformer. Under this approach, more aggressive PD techniques (for example, DC with a relative small buffer size) can be used to find the best conformers from an ensemble of available conformers. A more aggressive PD technique may yield smaller sub-molecules, which in turn may mean that fewer quantum resources may be required to conduct the experiment for a large molecule. This approach may thereby enable highly efficient and accurate predictions of the most stable conformers of a chemical system using quantum computing resources.

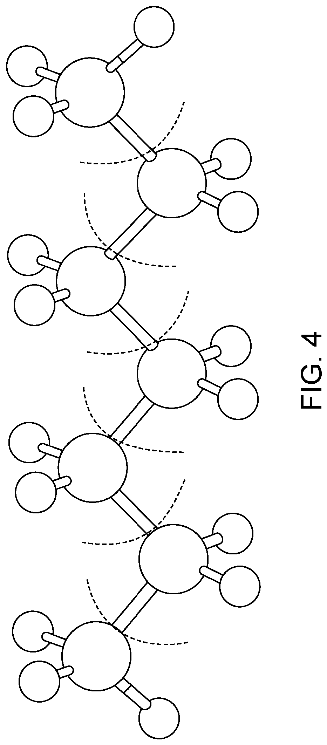

In this example, n-heptane was targeted, as shown in **FIG. 4,** where the dotted lines indicate the bond detached atom in the fragment molecular orbital (FMO) fragmentation. An ensemble of 40 conformations of n-heptane were generated by varying the four dihedral angles by 120 degrees (trans, gauche, gauche') and then removing symmetrically redundant conformations and high-energy conformations. In order to obtain the correlation between the total energies with and without problem decompositions (PD), CCSD was performed as a baseline reference and two problem decomposition methods, DC-CCSD and FMO-CCSD, were applied to this molecular system. Seven fragments were considered: 2 terminal $CH_3$ groups and 5 $CH_2$ groups. For DC, the buffer sizes of 3 Å, 4 Å, 5 Å, and 6 Å were examined. For FMO, the 2-body and 3-body expansions were examined. All calculations were performed using GAMESS-US with 6-31G basis set. The GAMESS quantum chemistry package was described in [Schmidt et al., "General Atomic and Molecular Electronic Structure System," Journal of Computational Chemistry, 1993, 14, 1347-1363]. The DC method was tested with a buffer size smaller than 3 Å, but the calculations for nearly all the conformers failed to converge to solutions.

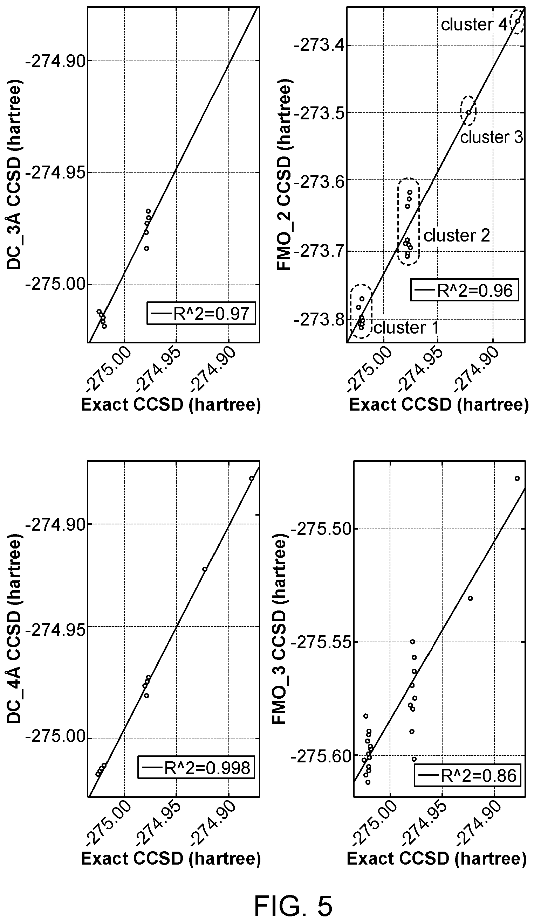

**FIG. 5** shows comparisons (list of conformer quantum mechanical energy values) between the exact CCSD and the DC-CCSD results, and between the exact CCSD and the FMO-CCSD results, for n-heptane. Good correlation was obtained between the results from the exact CCSD and from CCSD with problem decomposition; the coefficients of determination $R^2$ were more than 0.96 except for FMO with 3-body expansion (FMO_3). Although DC-CCSD provides better results, the DC calculations sometimes experienced difficulty with converging to a solution. For n-heptane, FMO provided a solution for all the 40 conformers examined, while DC provided a solution for 35 and 36 conformers using 3 Å and 4 Å buffer sizes, respectively. It is also noted that the number of spin orbitals required to solve one fragment can differ in the case of DC calculations, depending on the conformation, because the buffer region is defined based on the distance from the center of the fragment.

Referring again to **FIG. 5,** several clusters of conformers were observed, for example, as indicated by the dotted circles in the top right panel. Here, why these clusters are observed is briefly discussed. First, the relation between the exact CCSD energy and the diameter of the minimum sphere that can accommodate the conformer was examined. This diameter can be considered as a measure of the structural compactness of the conformer. As shown in the middle panel in **FIG. 6,** the total quantum mechanical energy generally increases when the conformer becomes structurally compact due to steric repulsions. However, as seen, the diameter does not fully explain the clustering of the conformers in terms of total quantum mechanical energy. Next, the relation between the total quantum mechanical energy and the distance between the two outermost carbon atoms in a dihedral (1-4 distance) was examined. As illustrated by the right panel in **FIG. 6,** which shows the relation between the total quantum mechanical energy and the smallest 1-4 distance for each conformer, the 1-4 distance explains the clustering behavior very well. The 1-4 distance varies depending on the dihedral angles being trans, gauche, or gauche'. In the case of trans, the 1-4 distance becomes the longest. The dihedral angles of gauche and gauche' have the same 1-4 distance which is shorter than the 1-4 distance of trans, causing the higher (less stable) total quantum mechanical energy due to steric repulsions. This is the main source of the discretization of total energies, and is the reason for the observation of clustering of conformers in terms of total quantum mechanical energy in the present molecular system.

## Example 2 (3-Methylheptane)

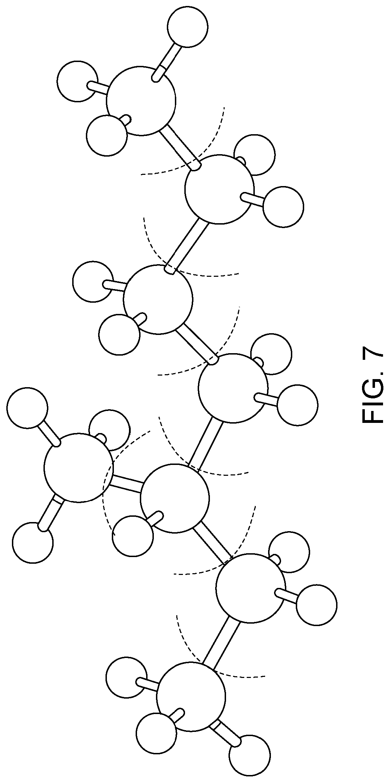

As observed, both FMO and DC work relatively well for a simple polymer system. Next, a diversified energy landscape was generated for examination by grafting one methyl group to the carbon atom at the "3" position of n-heptane, yielding 3-methylheptane, as shown in **FIG. 7.** The introduction of a methyl group to the "3" position renders the molecule asymmetric. As in the case of n-heptane, the ensemble of the conformations was generated for 3-methylheptane by varying the four dihedral angles by 120 degree (trans, gauche, gauche'), and 65 conformations were obtained after removing high-energy conformations.

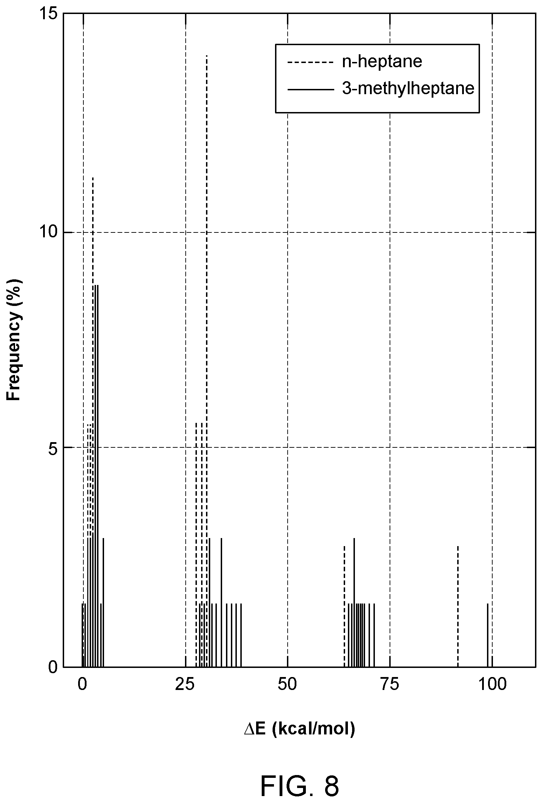

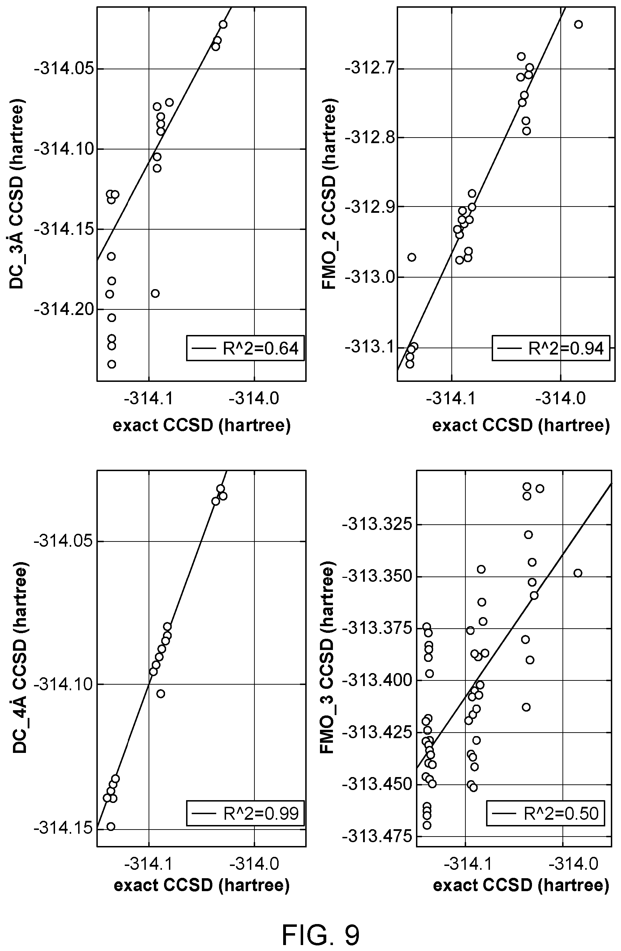

**FIG. 8** shows the quantum mechanical energy distribution (energies relative to the lowest one) obtained by CCSD to illustrate how one methyl group modulates and diversifies the quantum mechanical energy landscape from that of n-heptane. **FIG. 9** shows comparisons (list of quantum mechanical conformer energy values) between the exact CCSD and the DC-CCSD results, and between the exact CCSD and the FMO-CCSD results, for 3-methylheptane. As shown, the FMO (2-body) approach on 3-methylheptane exhibits stable performance, with an $R^2$ of 0.94. Although the total energies obtained by DC with 3Å buffer are somewhat closer to the exact CCSD than those obtained by FMO 2-body, the $R^2$ is lower than that of FMO. The DC approach on 3-methylheptane provides excellent agreement with exact CCSD when the buffer is increased to 4 Å. However, it is noted that DC again suffers slightly from the convergence failure issue. FMO provided a solution to all of the 65 conformers examined, while DC was able to provide a solution for 38 and 46 conformers with buffer sizes of 3 Å and 4 Å respectively.

## Computer systems

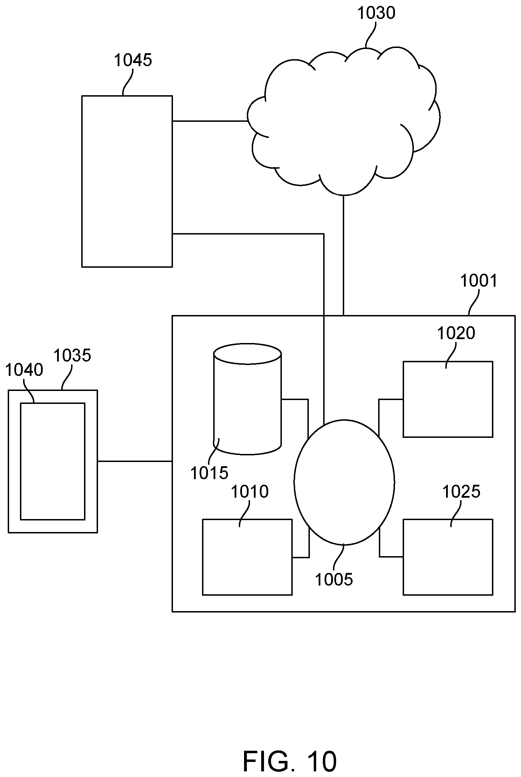

The present disclosure provides computer systems that are programmed to implement methods of the disclosure. **FIG. 10** shows a computer system **1001** that is programmed or otherwise configured to: determine an ensemble of conformations of a chemical system; decompose at least one conformation within the ensemble into a plurality of molecular fragments; determine, using a hybrid computing system, quantum mechanical energies and/or electronic structures of each of at least a subset of said plurality of molecular fragments; combine said determined quantum mechanical energies and/or electronic structures; and electronically output a report indicative of said combined quantum mechanical energies and/or electronic structures.

The computer system **1001** can regulate various aspects of methods and systems of the present disclosure, such as, for example, determining an ensemble of conformations of a chemical system; decomposing at least one conformation within the ensemble into a plurality of molecular fragments; determining, using a hybrid computing system, quantum mechanical energies and/or electronic structures of each of at least a subset of said plurality of molecular fragments; combining said determined quantum mechanical energies and/or electronic structures; and electronically outputting a report indicative of said combined quantum mechanical energies and/or electronic structures.

The computer system **1001** can be an electronic device of a user or a computer system that is remotely located with respect to the electronic device. The electronic device can be a mobile electronic device. The computer system **1001** includes a central processing unit (CPU, also "processor" and "computer processor" herein) **1005,** which can be a single core or multi core processor, or a plurality of processors for parallel processing. The computer system **1001** also includes memory or memory location **1010** (e.g., random-access memory, read-only memory, flash memory), electronic storage unit **1015** (e.g., hard disk), communication interface **1020** (e.g., network adapter) for communicating with one or more other systems, and peripheral devices **1025,** such as cache, other memory, data storage and/or electronic display adapters. The memory **1010,** storage unit **1015,** interface **1020** and peripheral devices **1025** are in communication with the CPU **1005** through a communication bus (solid lines), such as a motherboard. The storage unit **1015** can be a data storage unit (or data repository) for storing data. The computer system **1001** can be operatively coupled to a computer network ("network") **1030** with the aid of the communication interface 1020. The network **1030** can be the Internet, an internet and/or extranet, or an intranet and/or extranet that is in communication with the Internet.

The network **1030** in some cases is a telecommunication and/or data network. The network **1030** can include one or more computer servers, which can enable distributed computing, such as cloud computing. For example, one or more computer servers may enable cloud computing over the network **1030** ("the cloud") to perform various aspects of analysis, calculation, and generation of the present disclosure, such as, for example, determining an ensemble of conformations of a chemical system; decomposing at least one conformation within the ensemble into a plurality of molecular fragments; determining, using a hybrid computing system, quantum mechanical energies and/or electronic structures of each of at least a subset of said plurality of molecular fragments; combining said determined quantum mechanical energies and/or electronic structures; and electronically outputting a report indicative of said combined quantum mechanical energies and/or electronic structures. Such cloud computing may be provided by cloud computing platforms such as, for example, Amazon Web Services (AWS), Microsoft Azure, Google Cloud Platform, and IBM cloud. The network **1030,** in some cases with the aid of the computer system **1001,** can implement a peer-to-peer network, which may enable devices coupled to the computer system **1001** to behave as a client or a server. 'Cloud' services (including with one or more of the cloud platforms mentioned above) may also be used to provide data storage.

The CPU **1005** can execute a sequence of machine-readable instructions, which can be embodied in a program or software. The instructions may be stored in a memory location, such as the memory **1010.** The instructions can be directed to the CPU **1005,** which can subsequently program or otherwise configure the CPU **1005** to implement methods of the present disclosure. Examples of operations performed by the CPU **1005** can include fetch, decode, execute, and writeback.

The CPU **1005** can be part of a circuit, such as an integrated circuit. One or more other components of the system 1001 can be included in the circuit. In some cases, the circuit is an application specific integrated circuit (ASIC). The CPU **1005** may comprise one or more general purpose processors, one or more graphics processing units (GPUs), or a combination thereof.

The storage unit **1015** can store files, such as drivers, libraries and saved programs. The storage unit **1015** can store user data, e.g., an ensemble of conformation of the chemical system, a plurality of decomposed molecular fragments, quantum mechanical energies and/or electronic structures of molecular fragments, combined quantum mechanical energies and/or electronic structures of conformers, lists of molecular fragments with quantum mechanical energies and/or electronic structures, lists of conformers of a molecule with combined quantum mechanical energies and/or electronic structures, and reports indicative of combined quantum mechanical energies and/or electronic structures (sometimes exchanging data with the memory). The computer system **1001** in some cases can include one or more additional data storage units that are external to the computer system **1001,** such as located on a remote server that is in communication with the computer system **1001** through an intranet or the Internet.

The computer system **1001** can communicate with one or more remote computer systems through the network **1030.** For instance, the computer system **1001** can communicate with a remote computer system of a user. Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system **1001** via the network **1030.** The user may control or regulate various aspects of methods and systems of the present disclosure, such as, for example, determining an ensemble of conformations of a chemical system; decomposing at least one conformation within the ensemble into a plurality of molecular fragments; determining, using a hybrid computing system, quantum mechanical energies and/or electronic structures of each of at least a subset of said plurality of molecular fragments; combining said determined quantum mechanical energies and/or electronic structures; and electronically outputting a report indicative of said combined quantum mechanical energies and/or electronic structures.

Methods as described herein can be implemented by way of machine (e.g., computer processor) executable code stored on an electronic storage location of the computer system **1001,** such as, for example, on the memory **1010** or electronic storage unit **1015.** The machine executable or machine readable code can be provided in the form of software. During use, the code can be executed by the processor **1005.** In some cases, the code can be retrieved from the storage unit **1015** and stored on the memory **1010** for ready access by the processor **1005.** In some situations, the electronic storage unit **1015** can be precluded, and machine-executable instructions are stored on memory **1010.**

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 1001, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory, Solid-state memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system **1001** can include or be in communication with an electronic display **1035** that comprises a user interface (UI) **1040** for providing, for example, user selection of an ensemble of conformations of a chemical system; conformations within the ensemble for decomposing into a plurality of molecular fragments; at least a subset of said plurality of molecular fragments for determining quantum mechanical energies and/or electronic structures; and use of the Born-Oppenheimer approximation. Examples of UI's include, without limitation, a graphical user interface (GUI) and web-based user interface.

The computer system **1001** can include or be in communication with a non-classical computer (e.g., a quantum computer) **1045** for performing, for example, quantum algorithms (e.g., quantum mechanical energy and/or electronic structure calculations). The non-classical computer **1045** may be operatively coupled with the central processing unit **1005** and/or the network **1030** (e.g., the cloud).

Computer systems of the present disclosure may be as described, for example, in International Application No. PCT/CA2017/050709, U.S. Application No. 15/486,960, U.S. Patent No. 9,537,953 and U.S. Patent No. 9,660,859.

Methods and systems of the present disclosure can be implemented by way of one or more algorithms. An algorithm can be implemented by way of software upon execution by the central processing unit **1005.** The algorithm can, for example, determine an ensemble of conformations of a chemical system; decompose at least one conformation within the ensemble into a plurality of molecular fragments; determine, using a hybrid computing system, quantum mechanical energies and/or electronic structures of each of at least a subset of said plurality of molecular fragments; combine said determined quantum mechanical energies and/or electronic structures; and electronically output a report indicative of said combined quantum mechanical energies and/or electronic structures.

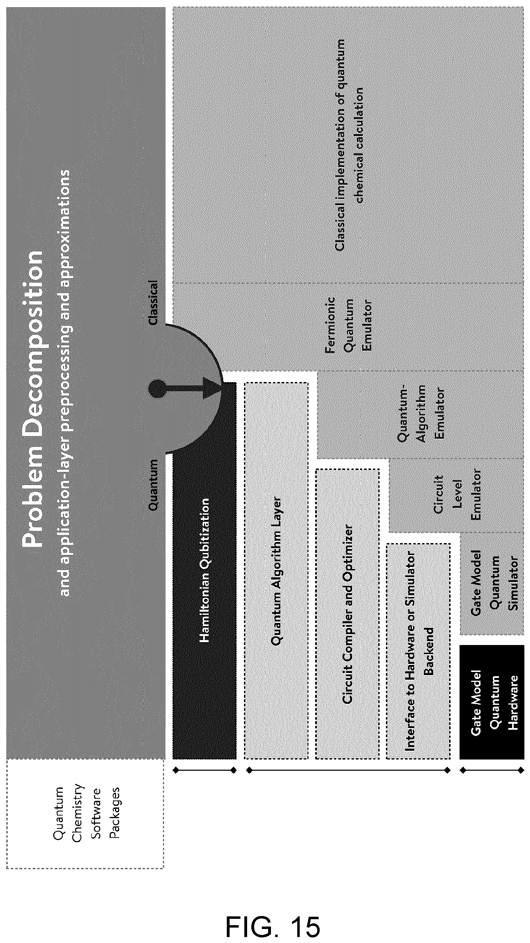

Though described herein with respect to certain systems, such as hybrid or quantum-classical computing or computing hardware, the problems of the present disclosure (such as a quantum chemistry problem or simulation) may be solved using a computing system comprising various types or combinations of systems, such as, for example, one or more classical computers, one or more non-classical computers (such as one or more quantum computers), or a combination of one or more classical computers and one or more non-classical computers. For instance, **FIG. 15** shows examples of systems or combinations of systems that may be used to solve problems of the present disclosure, such as a quantum chemistry problem or simulation.

It is intended that the following claims define the scope of the invention and that methods and structures within the scope of these claims be covered thereby.

### Claims

1. A method for performing a quantum mechanical energy or electronic structure calculation for a chemical system, said method being implemented by a hybrid computing system comprising at least one classical computer and at least one non-classical computer, said method comprising:

   (a) determining an ensemble of conformations of said chemical system;
   (b) decomposing at least one conformation within said ensemble into a plurality of molecular fragments;
   (c) determining, using said hybrid computing system, quantum mechanical energies or electronic structures of at least a subset of said plurality of molecular fragments;
   (d) combining said quantum mechanical energies or electronic structures determined in (c);
   (e) electronically outputting a report indicative of said quantum mechanical energies or electronic structures combined in (d); and
   (f) sorting the conformations by increasing or decreasing order of stability based on the combined quantum mechanical energies or electronic structures.

2. The method of claim 1, wherein said at least one non-classical computer comprises at least one quantum computer, and optionally wherein said at least one quantum computer comprises one or more members selected from the group consisting of: a quantum hardware device and a classical simulator of a quantum circuit.

3. The method of claim 1, wherein a given energy of said quantum mechanical energies comprises nuclear-nuclear repulsion energy.

4. The method of claim 1, further comprising providing an input to said hybrid computing system, said input comprising a set of atomic coordinates for said chemical system.

5. The method of claim 1, further comprising performing (b)-(d) for two or more conformations within said ensemble of conformations of said chemical system, optionally further comprising sorting said combined quantum mechanical energies or electronic structures of said at least said subset of said plurality of molecular fragments.

6. The method of claim 1, wherein (b) comprises applying one or more members selected from the group consisting of a fragment molecular orbital (FMO) method, a divide-and-conquer (DC) method, a density matrix embedding theory (DMET) method, a density matrix renormalization group (DMRG) method, a tensor network, and a method of increments.

7. The method of claim 1, wherein (c) comprises:

(a) determining a fermionic Hamiltonian of a given molecular fragment of said at least said subset of said plurality of molecular fragments;

(b) transforming said fermionic Hamiltonian into an equivalent qubit Hamiltonian;

(c) transforming said qubit Hamiltonian into a quantum circuit; and

(d) determining, using said quantum circuit, a quantum mechanical energy or electronic structure of said given molecular fragment.

8. The method of claim 7, further comprising determining said quantum mechanical energy or electronic structure using a molecular Hamiltonian or an electronic Hamiltonian.

9. The method of claim 7, wherein transforming said fermionic Hamiltonian into an equivalent qubit Hamiltonian comprises transforming a fermionic operator of a Hamiltonian to a qubit operator.

10. The method of claim 1, further comprising performing an *ab initio* molecular dynamics (AIMD) simulation of said chemical system.

11. The method of claim 10, wherein said AIMD simulation comprises:

prior to (a), obtaining an indication of a chemical system, said indication comprising coordinates of each particle of a plurality of particles in said chemical system and velocities of each particle in said chemical system; and subsequent to (d):

(i) determining, from said combined energy or electronic structure, a force on each particle in said chemical system;

(ii) updating said coordinates of said each particle in said chemical system and said velocities of said each particle in said chemical system; and

(iii) electronically outputting a report indicative of said coordinates or said velocities.

12. The method of claim 11, wherein (i) comprises applying Jordan's quantum algorithm for numerical gradient estimation to said quantum mechanical energy or electronic structure.

13. The method of claim 11, wherein (ii) comprises applying one or more members selected from the group consisting of a Verlet procedure, a velocity Verlet procedure, symplectic integration, Runge-Kutta intergration, and Beeman integration.

14. A system for performing a quantum mechanical energy or electronic structure calculation for a chemical system, comprising:

memory comprising instructions for performing said quantum mechanical energy or electronic structure calculation for said chemical system; and

a hybrid computing system operatively coupled to said memory, wherein said hybrid computing system comprises at least one classical computer and at least one non-classical computer, wherein said hybrid computing system is configured to execute said instructions to at least:

(a) determine an ensemble of conformations of said chemical system;

(b) decompose at least one conformation within said ensemble into a plurality of molecular fragments;

(c) determine quantum mechanical energies or electronic structures of at least a subset of said plurality of molecular fragments;

(d) combine said quantum mechanical energies or electronic structures determined in (c);

(e) electronically output a report indicative of said quantum mechanical energies or electronic structures combined in (d); and

(f) sorting the conformations by increasing or decreasing order of stability based on the combined quantum mechanical energies or electronic structures.

15. A non-transitory computer readable medium comprising machine-executable code that upon execution by a hybrid computing system comprising at least one classical computer and at least one non-classical computer, implements a method for performing a quantum mechanical energy or electronic structure calculation for a chemical system, said method comprising:

(a) determining an ensemble of conformations of said chemical system;
(b) decomposing at least one conformation within said ensemble into a plurality of molecular fragments;
(c) determining quantum mechanical energies or electronic structures of at least a subset of said plurality of molecular fragments;
(d) combining said quantum mechanical energies or electronic structures determined in (c);
(e) electronically outputting a report indicative of said quantum mechanical energies or electronic structures combined in (d); and
(f) sorting the conformations by increasing or decreasing order of stability based on the combined quantum mechanical energies or electronic structures.

**Patentansprüche**

1. Verfahren zum Ausführen einer quantenmechanischen Energie- oder Elektronenstrukturberechnung für ein chemisches System, wobei das Verfahren durch ein Hybridcomputersystem implementiert wird, das wenigstens einen klassischen Computer und wenigstens einen nicht klassischen Computer umfasst, wobei das Verfahren Folgendes umfasst:

(a) Bestimmen eines Ensembles von Konformationen des chemischen Systems;
(b) Zerlegen wenigstens einer Konformation in dem Ensemble in mehrere Molekularfragmente;
(c) Bestimmen quantenmechanischer Energien oder Elektronenstrukturen wenigstens einer Teilmenge der mehreren Molekularfragmente unter Verwendung des Hybridcomputersystems;
(d) Kombinieren der in (c) bestimmten quantenmechanischen Energien oder Elektronenstrukturen;
(e) elektronisches Ausgeben eines Berichts, der die in (d) kombinierten quantenmechanischen Energien oder Elektronenstrukturen angibt; und
(f) Sortieren der Konformationen nach aufsteigender oder absteigender Reihenfolge der Stabilität auf der Grundlage der kombinierten quantenmechanischen Energien oder Elektronenstrukturen.

2. Verfahren nach Anspruch 1, wobei der wenigstens eine nicht klassische Computer wenigstens einen Quantencomputer umfasst und wobei der wenigstens eine Quantencomputer optional ein oder mehrere Elemente umfasst, die aus der Gruppe gewählt sind, die aus Folgendem besteht: einer Quantenhardwarevorrichtung und einem klassischen Simulator einer Quantenschaltung.

3. Verfahren nach Anspruch 1, wobei eine gegebene Energie der quantenmechanischen Energien die internukleare Abstoßungsenergie umfasst.

4. Verfahren nach Anspruch 1, das ferner das Bereitstellen einer Eingabe in das Hybridcomputersystem umfasst, wobei die Eingabe eine Menge von Atomkoordinaten des chemischen Systems umfasst.

5. Verfahren nach Anspruch 1, das ferner das Ausführen von (b)-(d) für zwei oder mehr Konformationen in dem Ensemble von Konformationen des chemischen Systems umfasst, wobei es optional ferner das Sortieren der kombinierten quantenmechanischen Energien oder Elektronenstrukturen der wenigstens einen Teilmenge der mehreren Molekularfragmente umfasst.

6. Verfahren nach Anspruch 1, wobei (b) das Anwenden eines oder mehrerer Elemente umfasst, die aus der Gruppe gewählt sind, die aus einem Fragment-Molekülorbital-Verfahren (FMO-Verfahren), aus einem Teile-und-Herrsche-Verfahren (DC-Verfahren), aus einem Dichtematrix-Einbettungstheorie-Verfahren (DMET-Verfahren), aus einem Dichtematrix-Renormierungsgruppen-Verfahren (DMRG-Verfahren), aus einem Tensornetz und aus einem Inkrementverfahren besteht.

7. Verfahren nach Anspruch 1, wobei (c) Folgendes umfasst:

(a) Bestimmen eines Fermionen-Hamilton-Operators eines gegebenen Molekularfragments der wenigstens einen Teilmenge der mehreren Molekularfragmente;
(b) Transformieren des Fermionen-Hamilton-Operators zu einem äquivalenten Qubit-Hamilton-Operator;
(c) Transformieren des Qubit-Hamilton-Operators zu einer Quantenschaltung; und

(d) Bestimmen einer quantenmechanischen Energie- oder Elektronenstruktur des gegebenen Molekularfragments unter Verwendung der Quantenschaltung.

8. Verfahren nach Anspruch 7, das ferner das Bestimmen der quantenmechanischen Energie- oder Elektronenstruktur unter Verwendung eines Molekül-Hamilton-Operators oder eines Elektronen-Hamilton-Operators umfasst.

9. Verfahren nach Anspruch 7, wobei das Transformieren des Fermionen-Hamilton-Operators zu einem äquivalenten Qubit-Hamilton-Operator das Transformieren eines Fermionen-Hamilton-Operators in einen Qubit-Operator umfasst.

10. Verfahren nach Anspruch 1, das ferner das Ausführen einer Ab-initio-Moleküldynamik-Simulation (AIMD-Simulation) des chemischen Systems umfasst.

11. Verfahren nach Anspruch 10, wobei die AIMD-Simulation Folgendes umfasst:

vor (a) Erhalten einer Angabe eines chemischen Systems, wobei die Angabe Koordinaten jedes Teilchens mehrerer Teilchen in dem chemischen System und der Geschwindigkeiten jedes Teilchens in dem chemischen System umfasst; und
nach (d):

(i) Bestimmen einer Kraft auf jedes Teilchen in dem chemischen System aus der kombinierten Energie- oder Elektronenstruktur;
(ii) Aktualisieren der Koordinaten jedes Teilchens in dem chemischen System und der Geschwindigkeiten jedes Teilchens in dem chemischen System; und
(iii) elektronisches Ausgeben eines Berichts, der die Koordinaten oder die Geschwindigkeiten angibt.

12. Verfahren nach Anspruch 11, wobei (i) das Anwenden eines Jordanschen Quantenalgorithmus für die numerische Gradientenschätzung auf die quantenmechanische Energie- oder Elektronenstruktur umfasst.

13. Verfahren nach Anspruch 11, wobei (ii) das Anwenden eines oder mehrerer Elemente umfasst, die aus der Gruppe gewählt sind, die aus einer Verlet-Prozedur, aus einer Geschwindigkeits-Verlet-Prozedur, aus einer symplektischen Integration, aus einer Runge-Kutta-Integration und aus einer Beeman-Integration besteht.

14. System zum Ausführen einer quantenmechanischen Energie- oder Elektronenstrukturberechnung für ein chemisches System, wobei das System Folgendes umfasst:

einen Speicher, der Anweisungen zum Ausführen der quantenmechanischen Energie- oder Elektronenstrukturberechnung für das chemische System umfasst; und
ein Hybridcomputersystem, das mit dem Speicher funktional gekoppelt ist, wobei das Hybridcomputersystem wenigstens einen klassischen Computer und wenigstens einen nicht klassischen Computer umfasst, wobei das Hybridcomputersystem konfiguriert ist, die Anweisungen auszuführen, um wenigstens:

(a) ein Ensemble von Konformationen des chemischen Systems zu bestimmen;
(b) wenigsten eine Konformation in dem Ensemble in mehrere Molekularfragmente zu zerlegen;
(c) quantenmechanische Energien oder Elektronenstrukturen wenigstens einer Teilmenge der mehreren Molekularfragmente zu bestimmen;
(d) die in (c) bestimmten quantenmechanischen Energien oder Elektronenstrukturen zu kombinieren;
(e) elektronisch einen Bericht auszugeben, der die in (d) kombinierten quantenmechanischen Energien oder Elektronenstrukturen angibt; und
(f) Sortieren der Konformationen nach aufsteigender oder absteigender Reihenfolge der Stabilität auf der Grundlage der kombinierten quantenmechanischen Energien oder Elektronenstrukturen.

15. Nicht transitorisches computerlesbares Medium, das maschinenausführbaren Code umfasst, der bei Ausführung durch ein Hybridcomputersystem, das wenigstens einen klassischen Computer und wenigstens einen nicht klassischen Computer umfasst, ein Verfahren zum Ausführen einer quantenmechanischen Energie- oder Elektronenstrukturberechnung für ein chemisches System implementiert, wobei das Verfahren Folgendes umfasst:

(a) Bestimmen eines Ensembles von Konformationen des chemischen Systems;

(b) Zerlegen wenigstens einer Konformation in dem Ensemble in mehrere Molekularfragmente;

(c) Bestimmen quantenmechanischer Energien oder Elektronenstrukturen wenigstens einer Teilmenge der mehreren Molekularfragmente;

(d) Kombinieren der in (c) bestimmten quantenmechanischen Energien oder Elektronenstrukturen;

(e) elektronisches Ausgeben eines Berichts, der die in (d) kombinierten quantenmechanischen Energien oder Elektronenstrukturen angibt; und

(f) Sortieren der Konformationen nach aufsteigender oder absteigender Reihenfolge der Stabilität auf der Grundlage der kombinierten quantenmechanischen Energien oder Elektronenstrukturen.

## Revendications

1. Une méthode pour effectuer un calcul d'énergie mécanique ou de structure électronique quantique pour un système chimique, ladite méthode étant mise en œuvre par un système informatique hybride comprenant au moins un ordinateur classique et au moins un ordinateur non classique, ladite méthode comprenant le fait :

   (a) de déterminer un ensemble de conformations dudit système chimique ;
   (b) de décomposer au moins une conformation au sein dudit ensemble en une pluralité de fragments moléculaires ;
   (c) de déterminer, à l'aide dudit système informatique hybride, des énergies mécaniques ou structures électroniques quantiques d'au moins un sous-ensemble de ladite pluralité de fragments moléculaires ;
   (d) de combiner lesdites énergies mécaniques ou structures électroniques quantiques déterminées en (c) ;
   (e) de produire électroniquement en sortie un rapport indicatif desdites énergies mécaniques ou structures électroniques quantiques combinées en (d) ; et
   (f) de trier les conformations par ordre croissant ou décroissant de stabilité sur la base des énergies mécaniques ou structures électroniques quantiques combinées.

2. La méthode de la revendication 1, où ledit au moins un ordinateur non classique comprend au moins un ordinateur quantique, et facultativement où ledit au moins un ordinateur quantique comprend un ou plusieurs éléments sélectionnés dans le groupe constitué : d'un dispositif matériel quantique et d'un simulateur classique d'un circuit quantique.

3. La méthode de la revendication 1, où une énergie donnée desdites énergies mécaniques quantiques comprend de l'énergie de répulsion nucléaire-nucléaire.

4. La méthode de la revendication 1, comprenant en sus le fait de fournir une entrée audit système informatique hybride, ladite entrée comprenant un jeu de coordonnées atomiques pour ledit système chimique.

5. La méthode de la revendication 1, comprenant en sus le fait d'effectuer (b) à (d) pour deux ou plus de deux conformations au sein dudit ensemble de conformations dudit système chimique, facultativement comprenant en sus le fait de trier lesdites énergies mécaniques ou structures électroniques quantiques combinées dudit au moins dit sous-ensemble de ladite pluralité de fragments moléculaires.

6. La méthode de la revendication 1, où (b) comprend le fait d'appliquer un ou plusieurs éléments sélectionnés dans le groupe constitué d'une méthode FMO (*Fragment Molecular Orbital,* orbitales moléculaires de fragment), d'une méthode DC (*Divide-and-Conquer,* diviser et conquérir), d'une méthode DMET (*Density Matrix Embedding Theory,* théorie du plongement de la matrice densité), d'une méthode DMRG (*Density Matrix Renormalization Group,* groupe de renormalisation de la matrice densité), d'un réseau de tenseurs, et d'une méthode d'incréments.

7. La méthode de la revendication 1, où (c) comprend le fait :

   (a) de déterminer un hamiltonien fermionique d'un fragment moléculaire donné dudit au moins dit sous-ensemble de ladite pluralité de fragments moléculaires ;
   (b) de transformer ledit hamiltonien fermionique en un hamiltonien de qubits équivalent ;
   (c) de transformer ledit hamiltonien de qubits en un circuit quantique ; et
   (d) de déterminer, à l'aide dudit circuit quantique, une énergie mécanique ou structure électronique quantique dudit fragment moléculaire donné.

**8.** La méthode de la revendication 7, comprenant en sus le fait de déterminer ladite énergie mécanique ou structure électronique quantique à l'aide d'un hamiltonien moléculaire ou d'un hamiltonien électronique.

**9.** La méthode de la revendication 7, où le fait de transformer ledit hamiltonien fermionique en un hamiltonien de qubits équivalent comprend le fait de transformer un opérateur fermionique d'un hamiltonien en un opérateur de qubits.

**10.** La méthode de la revendication 1, comprenant en sus le fait d'effectuer une simulation AIMD (*Ab Initio Molecular Dynamics,* dynamique moléculaire *ab initio*) dudit système chimique.

**11.** La méthode de la revendication 10, où ladite simulation AIMD comprend le fait :

préalablement à (a), d'obtenir une indication d'un système chimique, ladite indication comprenant des coordonnées de chaque particule d'une pluralité de particules dans ledit système chimique et des vitesses de chaque particule dans ledit système chimique ; et
postérieurement à (d) :

(i) de déterminer, à partir de ladite énergie ou structure électronique combinée, une force sur chaque particule dans ledit système chimique ;
(ii) d'actualiser lesdites coordonnées de chaque dite particule dans ledit système chimique et lesdites vitesses de chaque dite particule dans ledit système chimique ; et
(iii) de produire électroniquement en sortie un rapport indicatif desdites coordonnées ou desdites vitesses.

**12.** La méthode de la revendication 11, où (i) comprend le fait d'appliquer l'algorithme quantique pour estimation de gradient numérique de Jordan à ladite énergie mécanique ou structure électronique quantique.

**13.** La méthode de la revendication 11, où (ii) comprend le fait d'appliquer un ou plusieurs éléments sélectionnés dans le groupe constitué d'une procédure de Verlet, d'une procédure de Verlet vitesse, d'une intégration symplectique, d'une intégration de Runge-Kutta, et d'une intégration de Beeman.

**14.** Un système pour effectuer un calcul d'énergie mécanique ou de structure électronique quantique pour un système chimique, comprenant :

une mémoire comprenant des instructions pour effectuer ledit calcul d'énergie mécanique ou de structure électronique quantique pour ledit système chimique ; et
un système informatique hybride couplé de manière fonctionnelle à ladite mémoire, où ledit système informatique hybride comprend au moins un ordinateur classique et au moins un ordinateur non classique, où ledit système informatique hybride est configuré pour exécuter lesdites instructions afin au moins :

(a) de déterminer un ensemble de conformations dudit système chimique ;
(b) de décomposer au moins une conformation au sein dudit ensemble en une pluralité de fragments moléculaires ;
(c) de déterminer des énergies mécaniques ou structures électroniques quantiques d'au moins un sous-ensemble de ladite pluralité de fragments moléculaires ;
(d) de combiner lesdites énergies mécaniques ou structures électroniques quantiques déterminées en (c) ;
(e) de produire électroniquement en sortie un rapport indicatif desdites énergies mécaniques ou structures électroniques quantiques combinées en (d) ; et
(f) de trier les conformations par ordre croissant ou décroissant de stabilité sur la base des énergies mécaniques ou structures électroniques quantiques combinées.

**15.** Un support lisible par ordinateur non transitoire comprenant du code exécutable par machine qui dès exécution par un système informatique hybride comprenant au moins un ordinateur classique et au moins un ordinateur non classique, met en œuvre une méthode pour effectuer un calcul d'énergie mécanique ou de structure électronique quantique pour un système chimique, ladite méthode comprenant le fait :

(a) de déterminer un ensemble de conformations dudit système chimique ;
(b) de décomposer au moins une conformation au sein dudit ensemble en une pluralité de fragments moléculaires ;
(c) de déterminer des énergies mécaniques ou structures électroniques quantiques d'au moins un sous-ensemble de ladite pluralité de fragments moléculaires ;

(d) de combiner lesdites énergies mécaniques ou structures électroniques quantiques déterminées en (c) ;

(e) de produire électroniquement en sortie un rapport indicatif desdites énergies mécaniques ou structures électroniques quantiques combinées en (d) ; et

(f) de trier les conformations par ordre croissant ou décroissant de stabilité sur la base des énergies mécaniques ou structures électroniques quantiques combinées.

100

Obtaining an indication of a molecule — 102

Generating or obtaining the list of conformers for the molecule — 104

Selecting the next conformer in the list — 106

Performing problem decomposition to populate a list of subsystems of the conformer — 108

Selecting the next subsystem in the list — 110

200

Calculating the energy and/or electronic structure of the subsystem — 112

Quantum chemistry simulator

Storing the resulting energy and/or electronic structure of the subsystem — 114

116

No — All subsystems of the conformer?

Yes

Combining all subsystem energies and/or electronic structures into the energy and/or electronic structure of the conformer — 118

120

No — All conformers of the molecule?

Yes

Sorting conformations based on the resulting energy and/or electronic structure of each conformer — 122

Providing an indication of the sorted list of conformers of the molecule based on resulting energy and/or electronic structure — 124

FIG. 1

FIG. 2

218

```
┌ ─ ─ ─ ─ ─ ─ ─ ─ ─ ─ ─ ─ ─ ─ ─ ─ ─ ─ ─ ─ ─ ─ ─ ─ ┐
│ ┌─────────────────────────────────────────┐       │
│ │ Translating the Hamiltonian into a quantum circuit │─310  │
│ └─────────────────────────────────────────┘       │
│                      │                              │
│                      ▼                              │
│ ┌─────────────────────────────────────────┐       │
│ │        Preparing the initial state        │─312  │
│ └─────────────────────────────────────────┘       │
│                      │                              │
│                      ▼                              │
│ ┌─────────────────────────────────────────┐       │
│ │ Performing quantum circuit optimization and │─314
│ │               compilation                 │       │
│ └─────────────────────────────────────────┘       │
│                      │                              │
│                      ▼                              │
│ ┌─────────────────────────────────────────┐       │
│ │ Obtaining the measurement of the resulting state │─318 │
│ └─────────────────────────────────────────┘       │
└ ─ ─ ─ ─ ─ ─ ─ ─ ─ ─ ─ ─ ─ ─ ─ ─ ─ ─ ─ ─ ─ ─ ─ ─ ┘
```

316

Quantum Hardware

## FIG. 3

FIG. 4

FIG. 5

FIG. 6

FIG. 7

FIG. 8

FIG. 9

FIG. 10

1300

| Obtaining an indication of a molecule | 1102 |

↓

| Incremental expansion of the energy | 1104 |

200

↓

| Solving the Schrödinger equation for each increment | 1106 |

| Quantum chemistry simulator |

↓

| Calculate the molecular electronic energy | 1108 |

## FIG. 11

• atoms

• fragments

• molecules

• molecules orbitals

## FIG. 12

1300

Obtaining an indication of molecular system —1302

Obtaining the initial coordinates of particles
in the system —1304

Obtaining the initial velocities of particles
in the system —1306

1308

Calculating the force on each particle of the system

1400

**Quantum-enabled
Problem
Decomposition
Toolkit**

Updating the coordinates and the velocities of the
particles in the system —1310

Storing the coordinates and the velocities of the
particles in the system —1312

1314

Yes

More steps?

No

Providing an indication of resulting trajectory
of molecular system —1316

FIG. 13

1400

Quantum-enabled Problem Decomposition for Electronic Structure Estimation

100

Force Estimation

1402

FIG. 14

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62593060
- US 20180107526 A
- CA 2017050709 W
- US 486960
- US 9537953 B
- US 9660859 B

**Non-patent literature cited in the description**

- **KITAURA et al.** Fragment molecular orbital method: an approximate computational method for large molecules. *Chemical Physics Letters*, 1999, vol. 313, 701
- **FEDOROV et al.** Exploring chemistry with the fragment molecular orbital method. *Physical Chemistry Chemical Physics*, 2012, vol. 14, 7562
- **YANG**. Direct calculation of electron density in density-functional theory: Implementation for benzene and a tetrapeptide. *Physical Review A*, 1991, vol. 44, 7823
- **AKAMA et al.** Implementation of divide-and-conquer method including Hartree-Fock exchange interaction. *Journal of Computational Chemistry*, 2007, vol. 28, 2003
- Divide-and-conquer approaches to quantum chemistry: Theory and implementation. **KOBAYASHI et al.** Linear-Scaling Techniques in Computational Chemistry and Physics: Methods and Applications. Springer, 2011, 97-127
- **KNIZIA et al.** Density Matrix Embedding: A Simple Alternative to Dynamical Mean-Field Theory. *Physics Review Letters*, 2012, vol. 109, 186404

- **WOUTERS et al.** A Practical Guide to Density Matrix Embedding Theory in Quantum Chemistry. *Journal of Chemical Theory and Computation*, 2016, vol. 12, 2706
- **ZIMMERMAN et al.** Strong Correlation in Incremental Full Configuration Interaction. *Journal of Chemical Physics*, 2017, vol. 146, 224104
- **ASPURU-GUZIK et al.** Simulated Quantum Computation of Molecular Energies. *Science*, 2005, vol. 309, 1704
- **MCCLEAN et al.** The theory of variational hybrid quantum-classical algorithms. *New Journal of Physics*, 2016, vol. 18, 023023
- **MATSUURA et al.** VanQver: The Variational and Adiabatically Navigated Quantum Eigensolver. *arXiv:1810.11511*, 31 October 2018
- **JORDAN**. Fast Quantum Algorithm for Numerical Gradient Estimation. *Physical Review Letters*, 2015, vol. 95, 050501
- **SCHMIDT et al.** General Atomic and Molecular Electronic Structure System. *Journal of Computational Chemistry*, 1993, vol. 14, 1347-1363